# EUROPÄISCHE PATENTSCHRIFT

(11) **EP 2 830 684 B1**
(45) Veröffentlichungstag und Bekanntmachung des Hinweises auf die Patenterteilung: **07.06.2017**
(21) Anmeldenummer: 13711419.5
(22) Anmeldetag: 22.03.2013
(51) Int. Cl.: A61M 5/20, A61M 5/24, A61M 5/31, A61M 5/315

(54) **INJEKTIONSVORRICHTUNG MIT EINEM DOSIERGLIED UND EINER VORGESPANNTEN AUSSCHÜTTFEDER**
INJECTION DEVICE WITH DOSE DISPLAY AND CLOCKWORK DRIVE
DISPOSITIF D'INJECTION AVEC AFFICHAGE DE DOSE ET ENTRAÎNEMENT PAR RESSORT

(30) Priorität: 30.03.2012 EP 12162777
(43) Veröffentlichungstag der Anmeldung: 04.02.2015
(73) Patentinhaber: TecPharma Licensing AG, 3401 Burgdorf (CH)
(72) Erfinder: SCHENKER, Susanne, 4900 Langenthal (CH); STREIT, Ursina, 3322 Schönbühl (CH)
(86) Internationale Anmeldenummer: PCT/EP2013/056095
(87) Internationale Veröffentlichungsnummer: WO 2013/144021

(56) Entgegenhaltungen:
- WO-A1-2008/087071
- WO-A1-2010/081489
- WO-A1-2010/115670
- WO-A1-2012/037938

## Beschreibung

Die Erfindung betrifft eine Injektionsvorrichtung zum Verabreichen eines flüssigen Produkts, insbesondere eines Medikaments, wie z. B. Insulin zur Diabetestherapie. Im Besonderen betrifft die Erfindung eine Antriebs- und Dosiervorrichtung für eine solche Injektionsvorrichtung. Eine Antriebs- und Dosiervorrichtung, wie es in dem Oberbegriff nach Anspruch 1 beschrieben ist, ist auch aus dem Dokument WO2012/037938 A1 bekannt. Aus dem Stand der Technik, nämlich der WO 2008/031237 A1 ist ein Injektionsgerät mit einer Dosisanzeigetrommel und einer Antriebsfeder bekannt. Die Antriebsfeder ist eine Uhrenfeder, die spiralförmig aus einem bandförmigen Material gewickelt ist. Beim Einstellen der gewünschten Produktdosis wird die Feder mit einer rotatorischen Bewegung gespannt. Für die Dosisausschüttung wird durch Betätigen eines Betätigungsknopfs am proximalen Ende der Vorrichtung eine Kolbenstange der Vorrichtung mit der Feder gekoppelt, wodurch die Feder die in ihr gespeicherte Energie an die Kolbenstange abgeben kann, wodurch die Kolbenstange in Ausschüttrichtung bewegt wird. Zum Einstellen einer neuen Dosis wird die Feder durch Drehen des Dosierknopfs wieder vorgespannt und so weiter. Dies wird so oft wiederholt, bis der Produktbehälter geleert ist.

Aus der US 5,104,380 A ist eine Injektionsvorrichtung mit einer Schraubenfeder bekannt, welche bei der Dosierung ebenfalls rotatorisch vorgespannt wird, so dass die Schraubenfeder als Torsionsfeder bezeichnet werden kann. Die vor jeder Produktausschüttung durch Drehen eines Drehknopfs vorgespannte Feder gibt ihre Energie zum Vortrieb der Kolbenstange an die Kolbenstange ab.

Aus der WO 2006/77466 A2 ist eine Injektionsvorrichtung bekannt, die einen direkten mechanischen Antrieb zwischen der Person, welche die Injektionskraft aufbringt, und der Kolbenstange, die für die Injektion des Medikaments in distale Richtung verschoben wird, aufweist.

Es ist eine Aufgabe der Erfindung, eine Antriebs- und Dosiervorrichtung für eine Injektionsvorrichtung zur Verabreichung eines flüssigen Produkts anzugeben, die dem Verwender eine einfachere Verwendung der Vorrichtung, insbesondere eine einfachere Dosiseinstellung erlaubt.

Die Aufgabe wird durch die Merkmale des Anspruchs 1 gelöst. Vorteilhafte Weiterbildungen ergeben sich aus den abhängigen Ansprüchen, der Beschreibung und den Figuren.

Es ist eine Aufgabe, insbesondere in einer vorteilhaften Weiterbildung der Erfindung eine Antriebs- und Dosiervorrichtung für eine Injektionsvorrichtung mit einer verbesserten Dosisanzeige anzugeben und die dem Verwender der Vorrichtung insbesondere verbessert Aufschluss über den Betriebszustand der Vorrichtung gibt. Diese Aufgabe wird insbesondere mit den Merkmalen des Anspruchs 14 gelöst.

Die Erfindung geht von einem Antriebsmechanismus für eine Injektionsvorrichtung zum Verabreichen eines flüssigen Medikaments oder Produkts aus. Der Antriebsmechanismus weist ein Gehäuse auf. Das Gehäuse ist vorzugsweise hülsenförmig und/oder länglich ausgebildet. Das Gehäuse kann sich z. B. entlang seiner Längsachse erstrecken.

Das Gehäuse kann optional einen Produktbehälter aufnehmen oder selbst den Produktbehälter bilden. Das Gehäuse kann ein- oder mehrteilig sein. Z. B. kann das Gehäuse einen proximalen Gehäuseteil bilden, der die Antriebs- und Dosiervorrichtung umfasst oder aufweist. Das Gehäuse kann ferner einen Produktbehälterhalter aufweisen, der den Produktbehälter, wie z. B. eine Karpule, aufnimmt und mit dem Gehäuse oder dem proximalen Gehäuseteil verbunden ist. Diese Verbindung kann derart sein, dass der Produktbehälterhalter und das Gehäuse oder der proximale Gehäuseteil nach dem Verbinden unlösbar, d. h. nur durch Zerstörung von Verbindungselementen lösbar ist. Eine solche Lösung ist insbesondere bei Einweginjektionsvorrichtungen von Vorteil, die nach dem das in dem Produktbehälter enthaltene Produkt vollständig ausgeschüttet ist, als Ganzes entsorgt werden. Alternativ kann der Produktbehälterhalter auch lösbar an dem Gehäuse befestigt werden, wodurch es möglich ist, wenngleich auch weniger bevorzugt, die Antriebs- und Dosiervorrichtung gegebenenfalls mehrfach zu verwenden, d. h. einen leeren Produktbehälter gegen einen gefüllten Produktbehälter auszutauschen.

Das Gehäuse dient vorzugsweise dazu, vom Verwender der Vorrichtung ergriffen zu werden. Insbesondere kann das Gehäuse eine im Wesentlichen zylindrische Form aufweisen. Das Gehäuse kann z. B. eine Zeigeeinrichtung, insbesondere ein Fenster aufweisen, mittels der oder durch welches eine aktuell eingestellte Dosis, vorzugsweise von einer Skala eines Dosiseinstelleelements, ablesbar ist.

In einem ersten Aspekt, der zum Beispiel die Erfindung vorteilhaft weiterbildet, umfasst die Antriebs- und Dosiervorrichtung, die insbesondere zusammen mit dem Produktbehälter eine Injektionsvorrichtung bildet, neben einem Gehäuse ein Dosisanzeigeelement, über dessen Umfang eine Dosisskala angeordnet ist. Das Dosisanzeigeelement kann z. B. im Querschnitt ringförmig sein. Das Dosisanzeigeelement kann z. B. eine Dosisanzeigetrommel oder ein Dosisanzeigering sein. Die Dosisskala kann sich über den Umfang des Dosisanzeigeelements, vorzugsweise wendelförmig, erstrecken. Die Dosisskala umfasst vorzugsweise eine Vielzahl von Werten, die aneinandergereiht angeordnet sind und die Dosisskala ergeben. Vorzugsweise handelt es sich um Zahlenwerte, welche die gewünschte Produktdosis in internationalen Einheiten (I.U.) angeben.

Alternativ kann die Dosisskala ohne Steigung über den Umfang des Dosisanzeigeelements, wie z. B. des Dosisanzeigerings, angeordnet sein, wobei sich die Skalenwerte dann nach einer Umdrehung des Dosisanzeigeelements wiederholen. Bei einer Dosisskala mit Steigung, d. h. einer wendelförmigen Dosisskala kann das Dosisanzeigeelement, insbesondere die Dosisanzeigetrommel, mit mehr als einer Umdrehung gedreht werden, ohne dass sich die Skalenwerte wiederholen, wodurch vorteilhaft die Skalenwerte größer oder mehr Skalenwerte dargestellt werden können.

Die Antriebs- und Dosiervorrichtung umfasst ferner eine Zeigeeinrichtung, wobei das Dosisanzeigeelement zur Einstellung der zu verabreichenden Dosis relativ zu der Zeigeeinrichtung und insbesondere um eine Drehachse, die vorzugsweise der Längsachse der Antriebs- und Dosiervorrichtung oder/und des Dosisanzeigeelements entspricht, drehbar ist. Hierbei kann es sich um eine reine Drehbewegung, d. h. eine Drehbewegung ohne überlagerte Axialbewegung handeln. Vorzugsweise ist der Drehbewegung eine Axialbewegung überlagert, wodurch das Dosisanzeigeelement zur Einstellung der zu verabreichenden Dosis relativ zu der Zeigeeinrichtung schraubbar ist. Ein schraubbares Dosisanzeigeelement lässt sich vorteilhaft mit einer wendelförmigen Dosisskala kombinieren, wobei die Schraubbewegung und die Dosisskala vorteilhafterweise die gleiche Steigung aufweisen. Ein ohne Axialbewegung drehbares Dosisanzeigeelement lässt sich vorteilhaft mit einer steigungsfreien Dosisskala kombinieren.

Mittels der Zeigeeinrichtung, die bevorzugt an dem Gehäuse gebildet wird, ist ein Wert der Dosisskala ablesbar, welcher der eingestellten Dosis entspricht. Die Zeigeeinrichtung kann z. B. ein Fenster sein, das durch einen Durchbruch im Gehäuse oder durch einen transparenten Einsatz gebildet sein kann. Alternativ oder optional kann die Zeigeeinrichtung ein Pfeil sein oder einen Pfeil aufweisen, der z. B. zusätzlich zu dem Fenster den Wert der Dosisskala markiert, welcher der eingestellten Dosis entspricht. Dies ist z. B. dann von Vorteil, wenn in dem Fenster noch ein anderer Wert zumindest teilweise erscheint, um eine eindeutige Dosisauswahl sicherzustellen. Der Zeiger kann z. B. ein Vorsprung oder ein Aufdruck oder eine Kerbe oder dergleichen sein.

Die Antriebs- und Dosiervorrichtung umfasst ein Dosierglied, das z.B. als Dosierknopf ausgebildet ist und optional als Dosiseinstellglied bezeichnet werden kann. Das Dosierglied ist vorzugsweise vom Verwender (Patient, Arzt, medizinisches Hilfspersonal) der Antriebs- und Dosiervorrichtung greifbar und bildet vorzugsweise eine äußere, insbesondere von außen zugängliche Oberfläche der Antriebs- und Dosiervorrichtung. Zur Einstellung der auszuschüttenden oder zu verabreichenden Dosis wird das Dosierglied vorzugsweise vom Verwender ergriffen und relativ zu dem Gehäuse und insbesondere der Zeigeeinrichtung um eine Drehachse, die vorzugsweise der Längsachse der z.B. länglich ausgestalteten Antriebs- und Dosiervorrichtung entspricht, verdreht. Das Dosierglied ist vorzugsweise axialfest mit dem Gehäuse verbunden, insbesondere entlang einer Längsachse des Gehäuses verschiebefest, wodurch vorteilhaft die intuitive Handhabung der Vorrichtung durch den Verwender erleichtert wird, da er lediglich eine Drehbewegung des Dosierglieds zur Dosiseinstellung ausführen braucht.

Insbesondere kann das Dosisanzeigeelement zumindest während der Dosiseinstellung verdrehfest, aber z.B. axial verschiebbar mit dem Dosierglied verbunden oder gekoppelt sein. Für die intuitive Bedienung ist es vorteilhaft, wenn das Dosisanzeigeelement bei Drehung des Dosierglieds um einen Drehwinkel ebenfalls um diesen Drehwinkel verdreht wird.

Die Antriebs- und Dosiervorrichtung kann ein Betätigungsglied z.B. in der Gestalt eines Betätigungsknopfs aufweisen. Das Betätigungsglied kann eine äußere Oberfläche der Antriebs- und Dosiervorrichtung bilden und/oder von außen zugänglich sein. Das Betätigungsglied kann am proximalen, insbesondere hinteren Ende der Antriebs- und Dosiervorrichtung gebildet sein oder dieses Ende bilden. Das Betätigungsglied lässt sich auf diese Weise vorteilhaft mit dem Daumen der Hand, die das Gehäuse umgreift betätigen, insbesondere drücken. Durch Loslassen des Betätigungsglieds kann die Betätigung beendet werden. Unter "betätigen" wird die Verschiebung des Betätigungsglieds in die Antriebs- und Dosiervorrichtung, insbesondere in distale Richtung verstanden, wodurch insbesondere eine Produktausschüttung bewirkt werden kann. Das Betätigungsglied ist vorteilhaft relativ zu dem Dosierglied verschiebbar und kann insbesondere von dem Dosierglied axial verschiebbar aufgenommen sein.

Das Betätigungsglied kann vorteilhaft gegen die Kraft einer Feder, insbesondere einer Rücksetz- oder Kupplungsfeder verschiebbar, insbesondere betätigbar sein, wodurch diese Feder gespannt wird. Durch Loslassen kann diese Feder das Betätigungsglied zurücksetzen, insbesondere relativ zu dem Dosierglied, insbesondere in proximale Richtung oder aus der Antriebs- und Dosiervorrichtung heraus, verschieben.

Die Antriebs- und Dosiervorrichtung umfasst ferner ein Lagerelement, mit dem das Dosisanzeigeelement in einem Eingriff ist. Dieser Eingriff bewirkt vorteilhaft die Dreh- oder Schraubbewegung des Dosisanzeigeelements relativ zu der Zeigeeinrichtung. Z. B. kann der Eingriff zwischen Dosisanzeigeelement und Lagerelement ein Gewindeeingriff sein. Insbesondere kann das Lagerelement ein Außengewinde und das Dosisanzeigeelement ein Innengewinde aufweisen, wobei diese Gewinde ineinander greifen und dadurch bewirken, dass das Dosisanzeigeelement relativ zu dem Lagerelement schraubbar ist.

Insbesondere ist das Dosisanzeigeelement zwischen einer Maximaldosisposition und einer Nulldosisposition hin und her dreh- oder schraubbar. In der Nulldosisposition kann vorteilhaft die Dosis oder Ziffer "0" in der Zeigeeinrichtung ablesbar sein. In der Maximaldosisposition kann vorteilhaft die maximal mit der Antriebs- und Dosiervorrichtung ausschüttbare Produktdosis ablesbar sein.

In der Nulldosisposition kann das Dosisanzeigeelement gegen die Drehung in eine Drehrichtung gesperrt sein, nämlich in die Drehrichtung, die bewirken würde, dass eine Dosis kleiner als null eingestellt wird. In der Nulldosisposition kann das Dosisanzeigeelement vorzugsweise nur in die Drehrichtung bewegt werden, die eine Erhöhung der Dosis bewirkt. In der Maximaldosisposition ist das Dosisanzeigeelement vorzugsweise gegen die Drehung in eine Drehrichtung, nämlich in die Drehrichtung, die die Einstellung einer Dosis über die maximal einstellbare Dosis hinaus bewirken würde, blockiert. Das Dosisanzeigeelement kann in der Maximaldosisposition insbesondere nur in die Drehrichtung gedreht werden, die eine Verringerung der Produktdosis bewirkt.

Das Dosisanzeigeelement kann z. B. einen Anschlag aufweisen, der in der Nulldosisposition an einen Gegenanschlag anschlägt und somit die Drehung in eine Drehrichtung verhindert. Der gleiche oder ein zusätzlicher Anschlag des Dosisanzeigeelements kann die Drehung des Dosisanzeigeelements über die Maximaldosis hinaus verhindern. Insbesondere kann hierfür ein weiterer Gegenanschlag, nämlich ein Maximaldosisgegenanschlag vorgesehen sein. Dementsprechend kann der andere Gegenanschlag als Nulldosisgegenanschlag bezeichnet werden. Das Dosisanzeigeelement kann demnach einen Nulldosisanschlag für den Nulldosisgegenanschlag und einen Maximaldosisanschlag für den Maximaldosisgegenanschlag aufweisen. Vorzugsweise wirken der Anschlag oder die Anschläge in Umfangsrichtung und/oder in Axialrichtung.

Die Antriebs- und Dosiervorrichtung zeichnet sich in einem ersten Aspekt dadurch aus, dass das Lagerelement zusammen mit dem Dosisanzeigeelement relativ zu dem Gehäuse und entlang der Drehachse, insbesondere in distale Richtung verschiebbar ist. Dieser Aspekt kann die Antriebs- und Dosiervorrichtung nach einem zweiten hierin beschriebenen Aspekt vorteilhaft weiterbilden. Alternativ kann das Dosisanzeigeelement ein Gewinde aufweisen, welches im Eingriff mit dem Gehäuse ist. Dadurch lässt sich das Dosisanzeigeelement zwar relativ zu dem Gehäuse hin und her schrauben, aber nicht unabhängig von der Schraubbewegung mit einer insbesondere reinen Axialbewegung verschieben.

Vorzugsweise ist das Betätigungsglied so mit dem Lagerelement gekoppelt, dass eine Verschiebung des Betätigungsglieds relativ zu dem Gehäuse und/oder dem Dosierglied eine Verschiebung des Lagerelements relativ zu dem Gehäuse und/oder dem Dosierglied insbesondere entlang der Längsachse der Antriebs- und Dosiervorrichtung bewirkt.

Dadurch dass das Dosisanzeigeelement erfindungsgemäß in einem Eingriff mit dem Lagerelement ist und sich das Lagerelement relativ zu dem Gehäuse und entlang der Drehachse verschieben lässt, lässt sich auch das Dosisanzeigeelement relativ zu dem Gehäuse und entlang der Drehachse unabhängig von der Dreh- oder Schraubbewegung, welche das Dosisanzeigeelement bei der Dosiseinstellung ausführt, verschieben. Die Antriebs- und Dosiervorrichtung nach dem zweiten Aspekt lässt sich im Grunde aber auch vorteilhaft mit dem alternativen Dosisanzeigeelement kombinieren, das in dem Gewindeeingriff mit dem Gehäuse oder einem gehäusefesten Element ist. In dieser Alternative kann das Lagerelement von dem Gehäuse gebildet sein oder Teil des Gehäuses sein, wobei das Lagerelement dann z. B. dreh- und axialfest in Bezug auf das übrige Gehäuse sein kann.

Vorteilhaft kann an der Zeigeeinrichtung und/oder an dem Dosisanzeigeelement abgelesen werden, dass das Lagerelement zusammen mit dem Dosisanzeigeelement verschoben wurde. Hierdurch lässt sich durch den Verwender kontrollieren, in welchem Betriebszustand sich die Antriebs- und Dosiervorrichtung befindet, d. h. ob die Antriebs- und Dosiervorrichtung insbesondere das Betätigungsglied für eine Ausschüttung betätigt oder unbetätigt ist.

In einer bevorzugten Variante kann das Betätigungsglied oder/und das Lagerelement zusammen mit dem Dosisanzeigeelement relativ zu der Zeigeeinrichtung, dem Gehäuse und entlang der Drehachse verschiebbar sein. Im Bereich der Zeigeeinrichtung, insbesondere in dem Fenster der Zeigeeinrichtung kann eine von der Dosisskala verschiedene Markierung erscheinen, wenn das Lagerelement verschoben ist. Die Markierung ist vorzugsweise an dem Dosisanzeigeelement angeordnet. Wenn das Lagerelement unverschoben ist, insbesondere die Antriebs- und Dosiervorrichtung für die Produktausschüttung unbetätigt ist, kann die Markierung außerhalb der Zeigeeinrichtung angeordnet sein, wie z. B. von einem Gehäuse oder einem anderen Element verdeckt sein. Wird das Lagerelement verschoben, insbesondere die Antriebs- und Dosiervorrichtung für die Produktausschüttung betätigt, kann die Markierung aus dem abgedeckten Bereich hervortreten, so dass sie insbesondere an oder in der Zeigeeinrichtung erscheint oder ablesbar ist. Wird die Betätigung der Antriebs- und Dosiervorrichtung unterbrochen oder beendet, kann das Lagerelement in die Ursprungsposition zurückkehren, wodurch die Markierung vorzugsweise aus dem Bereich der Zeigeeinrichtung entfernt und insbesondere verdeckt wird.

In einer alternativen Variante kann das Betätigungsglied oder/und das Lagerelement zusammen mit dem Dosisanzeigeelement und der Zeigeeinrichtung relativ zu dem Gehäuse und entlang der Drehachse verschiebbar sein. Die Zeigeeinrichtung kann z. B. eine Blende sein oder zumindest die Funktion einer Blende erfüllen. Z. B. kann die Zeigeeinrichtung zumindest axial fest, vorzugsweise auch drehfest mit dem Lagerelement verbunden sein. Im Grunde kann das Lagerelement die Zeigeeinrichtung bilden. Selbstverständlich ist es aber auch möglich, dass die Zeigeeinrichtung ein von dem Lagerelement separates Teil ist. Die Zeigeeinrichtung kann z. B. hülsenförmig sein.

In dieser Variante kann die Verschiebung des Lagerelements bewirken, dass im Bereich der Zeigeeinrichtung eine von der Dosisskala verschiedene Markierung erscheint, die an oder auf der Zeigeeinrichtung angeordnet oder gebildet ist. Beispielsweise kann die Zeigeeinrichtung innerhalb des Gehäuses angeordnet sein. Die Markierung der Zeigeeinrichtung kann in unbetätigtem Zustand der Antriebs- und Dosiervorrichtung von dem Gehäuse oder einem anderem Element verdeckt sein. Wird die Antriebs- und Dosiervorrichtung, insbesondere das Betätigungsglied, betätigt, so dass das Dosisanzeigeelement zusammen mit der Zeigeeinrichtung verschoben wird, kann die Markierung aus seiner Abdeckung hervortreten, so dass die Markierung erblickbar oder ablesbar ist. Wird die Betätigung unterbrochen oder beendet, kann das Dosisanzeigeelement zusammen mit der Zeigeeinrichtung und dem Lagerelement in seine Ausgangsposition zurückverschoben werden, so dass die Markierung wieder unter der Abdeckung angeordnet ist.

Allgemein bevorzugt kann bei der Betätigung der Antriebs- und Dosiervorrichtung für eine Produktausschüttung eine Feder, insbesondere eine Kupplungs- oder Rückstellfeder gespannt werden. Mit anderen Worten ausgedrückt kann das Lagerelement bei der Betätigung gegen die Kraft einer insbesondere solchen Feder verschoben werden, insbesondere aus einer unbetätigten Position in eine betätigte Position. Die Feder kann z. B. eine Schrauben- oder Wendelfeder sein, die als Druckfeder wirkt. Diese Feder bewirkt ferner, dass beim Unterbrechen oder Beenden der Betätigung das Lagerelement in seine Ausgangsposition oder unbetätigte Position zurückgesetzt wird. Insbesondere wird das Lagerelement bei der Betätigung in distale Richtung verschoben. Mittels der Feder wird das Lagerelement in proximale Richtung zurück verschoben, wenn die Betätigung unterbrochen oder beendet wird.

Die Betätigung des Betätigungsglieds bewirkt insbesondere, dass das Lagerelement zusammen mit dem Dosisanzeigeelement relativ zu dem Gehäuse und entlang der Drehachse verschoben wird. Im weiteren Sinne kann die Betätigung des Betätigungsglieds bewirken, dass ein Vortriebsglied, dessen distales Ende vorgesehen ist, auf einen Kolben des an der Antriebs- und Dosiervorrichtung befestigten oder befestigbaren Produktbehälters zu wirken, in distale Richtung, insbesondere Ausschüttrichtung verschoben wird. Das Betätigungsglied kann z. B. an dem proximalen, d. h. hinteren Ende der Antriebs- und Dosiervorrichtung angeordnet sein oder das proximale Ende der Antriebs- und Dosiervorrichtung bilden. Alternativ kann das Betätigungselement seitlich am Gehäuse und/oder zwischen dem distalen Ende und dem proximalen Ende der Antriebs- und Dosiervorrichtung angeordnet sein. Allgemein kann das Betätigungsglied in der Art eines Betätigungsknopfs ausgebildet sein. Das Betätigungsglied wird bei der Betätigung vorzugsweise relativ zu dem Gehäuse oder dem Dosierglied verschoben. Insbesondere kann der Verwender der Vorrichtung das Betätigungsglied vorteilhaft z.B. mit dem Daumen seiner Hand, welche das Gehäuse der Antriebs- und Dosiervorrichtung umgreift, betätigen.

Das Betätigungsglied ist vorzugsweise mit dem Lagerelement so verbunden, dass es das Lagerelement bei Betätigung verschiebt, insbesondere über ein Kupplungsglied, das z. B. axialfest und drehbar mit dem Lagerelement verbunden sein kann.

In allgemein bevorzugten Ausführungen kann die Betätigung des Betätigungsglieds bewirken, dass das Dosisanzeigeelement relativ zu oder an dem Lagerelement oder dem Gehäuse gedreht, insbesondere geschraubt wird, insbesondere in eine Richtung, dass die sich bei der Drehbewegung an der Zeigeeinrichtung vorbei bewegenden Werte der Dosisskala zurückzählen. Vorzugsweise stehen der Drehwinkel des Dosisanzeigeelements und der Ausschütthub des Vortriebsglieds in einem proportionalen Zusammenhang, insbesondere zu jedem Zeitpunkt während der Dosisausschüttung. Hierdurch lässt sich eine Echtzeitanzeige verwirklichen, die bei der Dosisausschüttung zurückzählt, bis sie schließlich beim Wert 0 angelangt, wobei die Ausschüttung dieser Dosis dann beendet ist. Wird die Betätigung für die Ausschüttung während des Zurückdrehens des Dosisanzeigeelements unterbrochen, zeigt das Dosisanzeigeelement die noch für die Ausschüttung dieser Dosis erforderliche Restmenge an.

In einer Variante kann die Antriebs- und Dosiervorrichtung so ausgestaltet sein, dass die für das Zurückdrehen des Dosisanzeigeelements oder/und das Verschieben des Vortriebsglieds in distale Richtung benötigte Energie manuell, insbesondere durch eine auf das Betätigungsglied wirkende Kraft des Verwenders aufzubringen ist. Z. B. kann das Dosiseinstellglied, insbesondere der Dosierknopf aus dem proximalen Ende des Gehäuses für eine Dosiseinstellung herausgeschraubt werden, wobei er zur Dosisausschüttung unter Betätigung des Betätigungsglieds in das Gehäuse zurückgeschraubt wird.

In einer bevorzugten alternativen Variante kann die Antriebs- und Dosiervorrichtung so ausgestaltet sein, dass die für das Zurückdrehen des Dosisanzeigeelements oder/und das Verschieben des Vortriebsglieds in distale Richtung benötigte Energie automatisch, insbesondere durch eine in der Antriebs- und Dosiervorrichtung enthaltene Feder, insbesondere Ausschüttfeder, in der die benötigte Energie gespeichert ist oder werden kann, bereitgestellt wird. Z. B. kann die in der Ausschüttfeder gespeicherte Federenergie bei Betätigung des Betätigungsglieds an das Dosisanzeigeelement oder/und das Vortriebsglied abgegeben werden, so dass das Dosisanzeigeelement zurückgedreht und das Vortriebsglied in distale Richtung verschoben werden. Die Ausschüttfeder kann z. B. so mit dem Dosierglied gekoppelt sein, dass eine Drehung des Dosierglieds bei der Dosiseinstellung die Ausschüttfeder vorspannt. Die Feder kann dann die für die eingestellte Dosis erforderliche Energie speichern.

In einer bevorzugten Alternative kann die Feder bei Auslieferung der Antriebs- und Dosiervorrichtung bereits mit so viel Energie vorgespannt sein, dass die Energie für mehrere Ausschüttungen der Produktdosis ausreicht, insbesondere für die Ausschüttung des gesamten aus dem Produktbehälter ausschüttbaren Produkts ausreicht. In dieser Alternative kann das Dosierglied bei der Dosiseinstellung von der Feder entkoppelt sein, d.h. nicht so mit der Ausschüttfeder gekoppelt sein, dass eine Drehung des Dosierglieds ein Spannen der Feder bewirkt. Hierdurch lässt sich das Dosierglied für den Verwender bei der Dosiseinstellung mit deutlich weniger Kraftaufwand drehen.

Das Dosierglied, insbesondere der Dosierknopf kann das Betätigungsglied, insbesondere den Betätigungsknopf umgeben oder aufnehmen. Somit können das Dosierglied und das Betätigungsglied das proximale Ende der Antriebs- und Dosiervorrichtung bilden. Vorzugsweise ist das Betätigungsglied für die Betätigung relativ zu dem Dosierglied verschiebbar.

In Ausführungen, in denen die für die Ausschüttung benötigte Energie automatisch bereitgestellt wird, kann das Dosierglied vorzugsweise relativ zu, insbesondere an dem Gehäuse axialfest aber drehbar angeordnet sein.

In einem zweiten Aspekt, von dem insbesondere die vorliegende Erfindung ausgeht und der mit Merkmalen des ersten Aspekts kombinierbar ist, insbesondere mit oder ohne dem Merkmal, dass das Lagerelement zusammen mit dem Dosisanzeigeelement relativ zu dem Gehäuse und entlang der Drehachse verschiebbar ist, kann die Antriebs- und Dosiervorrichtung ein Vortriebsglied aufweisen, dessen distales Ende vorgesehen ist, auf einen Kolben, insbesondere mittelbar oder vorzugsweise unmittelbar zu wirken. Der Kolben kann Teil eines an der Antriebs- und Dosiervorrichtung befestigten oder befestigbaren Produktbehälters, wie z. B. einer Karpule sein. Das Vortriebsglied kann im weiteren Sinne als Kolbenstange bezeichnet werden, wobei das Vortriebsglied nicht notwendigerweise massiv sein muss, sondern auch hohl, wie z. B. hülsenförmig ausgestaltet sein kann. An dem distalen Ende des Vortriebsglieds kann optional ein z. B. drehbarer Flansch gebildet sein, der gegen den Kolben drückt. Allgemein ist es bevorzugt, dass das distale Ende des Vortriebsglieds gegen den Kolben drückt. Das Vortriebsglied ist vorzugsweise relativ zu dem Gehäuse entlang der Längsachse der Antriebs- und Dosiervorrichtung verschiebbar.

Die Antriebs- und Dosiervorrichtung kann ein Widerlager und vorzugsweise eine Führung aufweisen, wobei das Vortriebsglied relativ zu dem Widerlager und vorzugsweise auch relativ zu der Führung in eine Richtung, insbesondere in distale Richtung oder in Ausschüttrichtung, bewegbar ist, um eine Ausschüttung der eingestellten Produktdosis zu bewirken. Vorzugsweise kann das Vortriebsglied mittels oder an der Führung, entlang der Längsachse der Antriebs- und Dosiervorrichtung gerade oder axial geführt sein. Insbesondere kann das Vortriebsglied relativ zu dem Widerlager oder/und der Führung oder/und dem Gehäuse drehfest sein. In einer alternativen Ausführungsform kann das Vortriebsglied relativ zu dem Widerlager oder dem Gehäuse kombiniert mit einer Längsbewegung drehbar, d. h. relativ zu dem Widerlager schraubbar sein, wenngleich weniger bevorzugt. Allgemein kann die Führung oder/und das Widerlager von dem Gehäuse, insbesondere einem hülsenförmigen Gehäuseteil oder einem z.B. hülsenförmigen, gehäusefesten Element gebildet werden.

Das Vortriebsglied und die insbesondere von dem Gehäuse gebildete Führung können in einem Eingriff sein, der eine Drehung des Vortriebsglieds relativ zu dem Widerlager oder dem Gehäuse verhindert, aber eine Axialbewegung oder eine Schraubbewegung des Vortriebsglieds relativ zu dem Widerlager oder dem Gehäuse erlaubt. Die Führung kann z. B. eine Axialführung oder ein Gewinde mit einer nicht selbsthemmenden Gewindesteigung sein.

Die Führung oder der das Widerlager oder/und die Führung bildende Gehäuseabschnitt, insbesondere eine Innenhülse, kann vorzugsweise das Vortriebsglied umgeben, wie z. B. hülsenförmig umgeben und/oder gehäusefest sein oder von dem Gehäuse gebildet werden. Zwischen diesem hülsenförmigen Gehäuseteil und einem äußeren, vorzugsweise ebenfalls hülsenförmigen Gehäuseteil kann ein Ringspalt gebildet sein, der den Vorteil birgt, dass ein optional vorhandenes Dosisanzeigeelement, insbesondere Dosisanzeigetrommel, darin aufgenommen sein kann. Dies bewirkt, dass die Länge der Antriebs- und Dosiervorrichtung gering gehalten werden kann.

Nach dem zweiten Aspekt kann die Antriebs- und Dosiervorrichtung mindestens eine, wie z. B. genau eine, zwei oder drei zwischen dem Vortriebsglied oder einem Rotationsglied und dem Widerlager wirkende und insbesondere angeordnete Ausschüttfeder aufweisen. Die mindestens eine Feder kann sich z. B. an dem Vortriebsglied und/oder dem Widerlager abstützen. Beispielsweise kann sich die z. B. einzige Ausschüttfeder mit ihrem distalen Ende an dem Vortriebsglied und mit ihrem proximalen Ende an dem Widerlager abstützen. Insbesondere kann die mindestens eine Ausschüttfeder innerhalb der Führung oder des die Führung bildenden hülsenförmigen Gehäuseteils angeordnet sein. Ist das Vortriebsglied hülsenförmig, kann die mindestens eine Ausschüttfeder innerhalb des Vortriebsglieds angeordnet sein. Alternativ können eine erste Ausschüttfeder und eine zweite Ausschüttfeder insbesondere kinematisch zwischen dem Vortriebsglied und der Führung oder dem die Führung bildenden hülsenförmigen Gehäuseteil angeordnet sein. Die erste Ausschüttfeder kann z. B. die zweite Ausschüttfeder umgeben oder umgekehrt. Insbesondere kann die zweite Ausschüttfeder konzentrisch zur ersten Ausschüttfeder angeordnet sein. Die erste Ausschüttfeder und die zweite Ausschüttfeder können z. B. parallel oder in Serie geschaltet sein. Parallel geschaltete Ausschüttfedern bedeuten insbesondere, dass sich die erste und zweite Ausschüttfeder jeweils mit ihrem distalen Ende an dem Vortriebsglied und jeweils mit ihrem proximalen Ende an dem Widerlager abstützen können. Hierdurch lassen sich die Federkonstanten der ersten und zweiten Ausschüttfeder zu einer Gesamtfederkonstante addieren. In Serie geschaltete Ausschüttfedern bedeutet insbesondere, dass das distale Ende von einer aus erster und zweiter Ausschüttfeder gegen das proximale Ende der anderen aus erster und zweiter Ausschüttfeder drückt, insbesondere unmittelbar oder vorzugsweise mittelbar, wie z. B. über ein Zwischenglied. Z. B. stützt sich die erste Ausschüttfeder an dem Widerlager und dem Zwischenglied und die zweite Ausschüttfeder an dem Zwischenglied und dem Vortriebsglied ab. Beispielsweise kann das distale Ende der ersten Ausschüttfeder distal des proximalen Endes der zweiten Ausschüttfeder angeordnet sein. Aufgrund des Zwischenglieds kann die Federkraft der ersten Feder von ihrem distalen Ende auf das proximale Ende der zweiten Ausschüttfeder übertragen werden. Insbesondere kann das Zwischenglied hülsenförmig sein und in einem Ringspalt zwischen erster und zweiter Ausschüttfeder angeordnet sein. In Serie geschaltete Ausschüttfedern erlauben, über einen verhältnismäßig langen Federweg eine verhältnismäßig gleich bleibende Federkraft.

Z. B. kann die mindestens eine Ausschüttfeder, insbesondere die erste und zweite Ausschüttfeder eine Wendel- oder Schraubenfeder sein, die als Druckfeder oder als Torsionsfeder wirkt. Die mindestens eine Ausschüttfeder ist vorgespannt und wirkt so auf das Vortriebsglied, das sie versucht, das Vortriebsglied relativ zu dem Widerlager in distale Richtung, d. h. in Ausschüttrichtung zu verschieben. Die mindestens eine Ausschüttfeder ist im Auslieferungszustand der Antriebs- und Dosiervorrichtung insbesondere mit so viel Energie vorgespannt, dass sie die aus dem Produktbehälter maximal oder insgesamt ausschüttbare Produktmenge in mehreren Einzelausschüttungen d. h. insbesondere in mehreren Ausschüttungen einzelner Produktdosen ausschütten kann. Die Antriebs- und Dosiervorrichtung ist so ausgestaltet, dass nach jeder Einzelausschüttung oder Ausschüttung der Produktdosis, die nächste auszuschüttende Dosis neu eingestellt wird. Im Gegensatz zu Ausführungen, bei denen eine Ausschüttfeder bei jeder Dosiseinstellung neu vorgespannt wird, kann durch die mit der für die Ausschüttung der maximal aus dem Produktbehälter ausschüttbaren Produktmenge erforderlichen Energie vorgespannte Feder eine einfachere Dosiseinstellung erreicht werden, da das für die Dosiseinstellung relativ zu dem Gehäuse drehbare Dosierglied dann einfacher drehbar ist, weil die Feder bei der Dosiseinstellung nicht vorgespannt werden braucht. Dies erhöht den Anwendungskomfort für den Verwender der Vorrichtung.

Die Antriebs- und Dosiervorrichtung kann ferner ein Rotationsglied umfassen, dessen Drehung bewirkt, dass die Feder Energie an das Vortriebsglied abgibt, wodurch das Vortriebsglied in distale Richtung bewegt wird. Das Rotationsglied übernimmt vorzugsweise die Funktion eines Steuerglieds, wobei die Drehung des Rotationsglieds um einen bestimmten Drehwinkel den Vorschub des Vortriebsglieds um einen bestimmten Ausschütthub bewirkt. Durch selektive Freigabe oder Sperrung einer Drehung des Rotationsglieds relativ zu dem Gehäuse kann der Feder erlaubt werden, dass sie das Vortriebsglied relativ zu dem Widerlager in distale Richtung bewegen kann oder nicht bewegen kann. Insbesondere kann das Rotationsglied so mit dem Betätigungsglied gekoppelt sein, dass es bei der Betätigung des Betätigungsglieds für eine Produktausschüttung für eine Drehung relativ zu dem Gehäuse freigegeben ist und bei Nichtbetätigung des Betätigungsglieds für eine Rotation relativ zu dem Gehäuse gesperrt ist. Insbesondere kann zwischen Betätigungsglied und Rotationsglied eine Kupplung angeordnet sein, welche die Freigabe und Sperrung der Drehung des Rotationsglieds relativ zu dem Gehäuse bewirkt.

Vorteilhaft kann die Kupplung durch Betätigen des Betätigungsglieds die Drehung des Rotationsglieds relativ zu dem Gehäuse freigeben und durch Loslassen des Betätigungsglieds die Drehung des Rotationsglieds relativ zu dem Gehäuse blockieren.

Vorzugsweise ist die Ausschüttfeder kinematisch zwischen dem Kolben des Produktbehälters und dem Rotationsglied angeordnet. Hierdurch kann vermieden werden, dass die von der Ausschüttfeder bereitgestellte Ausschüttenergie zum großen Teil über das Rotationsglied laufen muss, wie es z. B. der Fall wäre, wenn das Rotationsglied kinematisch zwischen der Ausschüttfeder und dem Kolben angeordnet wäre. Dadurch lässt sich das Rotationsglied einfacher ausgestalten. Insbesondere kann somit erreicht werden, dass die mindestens eine Ausschüttfeder das Vortriebsglied antreibt und das Vortriebsglied das Rotationsglied antreibt.

Insbesondere kann das Vortriebsglied kinematisch zwischen der Ausschüttfeder und dem Rotationsglied angeordnet sein.

Insbesondere kann der Drehwinkel des Rotationsglieds proportional zu dem Ausschütthub des Kolbens oder des Vortriebsglieds sein. Dies lässt sich durch die selektive Sperrung oder Freigabe des Rotationsglieds erreichen.

Vorteilhaft kann das Rotationsglied in einem Eingriff, insbesondere einem Gewindeeingriff mit dem Vortriebsglied sein. Durch die Gewindesteigung dieses Gewindeeingriffs wird erreicht, dass z. B. bei einer vollständigen Umdrehung des Rotationsglieds relativ zu dem Gehäuse das Vortriebsglied von der Ausschüttfeder um einen Hub verschiebbar ist, welcher der Gewindesteigung entspricht.

Z. B. können das Rotationsglied eine Gewindestange und das Vortriebsglied eine Gewindemutter aufweisen oder sein, wobei das Gewinde der Gewindemutter in das Gewinde der Gewindestange eingreift.

In einem alternativen Beispiel können das Rotationsglied eine Gewindemutter und das Vortriebsglied eine Gewindestange aufweisen oder sein, wobei das Gewinde der Gewindemutter in das Gewinde der Gewindestange eingreift.

Vorzugsweise ist das Rotationsglied axial fest in Bezug auf das Gehäuse oder kann sich zumindest in eine, vorzugsweise in distaler Richtung axialfest an dem Gehäuse oder einem gehäusefesten Element, wie z. B. dem Widerlager abstützen.

Es ist vorteilhaft, dass das Rotationsglied während des Einstellens einer Dosis, d. h. im unbetätigten Zustand insbesondere mittels der Kupplung, drehfest mit dem Gehäuse verbunden ist und während der Betätigung der Vorrichtung zum Ausschütten der Produktdosis relativ zu dem Gehäuse gedreht wird oder drehbar ist. Die Antriebs- und Dosiervorrichtung kann eine Dosiseinstell- oder Anzeigeeinrichtung nach den Merkmalen a), b) und c) des Anspruchs 14 und vorteilhaft, aber nicht notwendigerweise, nach den Merkmalen d) und e) des Anspruchs 14 aufweisen. Das Dosisanzeigeelement, insbesondere die Dosisanzeigetrommel kann nämlich auch grundsätzlich in einem Gewindeeingriff mit dem Gehäuse oder einem gehäusefest angeordneten Element stehen.

Das Dosisanzeigeelement, insbesondere die Dosisanzeigetrommel kann während des Einstellens einer Dosis, d. h. im unbetätigten Zustand der Antriebs- und Dosiervorrichtung oder des Betätigungsglieds relativ zu dem Rotationsglied drehbar sein. Vorzugsweise ist das Dosisanzeigeelement während der Betätigung der Vorrichtung zum Ausschütten der Produktdosis relativ zu dem Rotationsglied drehfest und z.B. axial bewegbar, oder drehfest mit dem Rotationsglied verbunden, insbesondere mit der beschriebenen Kupplung oder einer weiteren Kupplung.

Vorteilhaft wird bewirkt, dass bei der Dosisausschüttung, d. h. bei der Betätigung des Betätigungsglieds, die Ausschüttfeder das Dosisanzeigeelement in seine Nulldosisposition zurückschraubt, insbesondere über das Rotationsglied und vorzugsweise über ein Kupplungsglied, welches vorzugsweise drehfest aber axial verschiebbar in Bezug auf das Dosisanzeigeelement angeordnet ist. Insbesondere können das Kupplungsglied und das Dosisanzeigeelement in einem drehfesten Eingriff sein, der eine Axialbewegung zwischen Dosisanzeigeelement und Kupplungsglied zulässt. Dieser Eingriff kann z. B. mittels einer Längsführung bewirkt werden. Bevorzugt ist das Kupplungsglied axial fest aber drehbar mit dem Lagerelement verbunden.

Beispielsweise kann die Antriebs- und Dosiervorrichtung eine erste Kupplungsstruktur, die in Bezug auf das Gehäuse drehfest ist, aufweisen. Das Rotationsglied kann eine zweite Kupplungsstruktur aufweisen oder bilden, die im Kupplungseingriff mit der ersten Kupplungsstruktur bewirkt, dass das Rotationsglied drehfest in Bezug auf das Gehäuse ist. Die erste Kupplungsstruktur kann z. B. von dem Gehäuse oder einem drehfest in Bezug auf das Gehäuse angeordneten aber axial verschiebbaren Element, wie z. B. dem Lagerelement oder einem insbesondere hülsenförmigen Kupplungsglied gebildet sein.

Das Kupplungsglied kann eine dritte Kupplungsstruktur aufweisen, die in einem Eingriff mit der ersten Kupplungsstruktur oder einer weiteren, vierten Kupplungsstruktur des Rotationsglieds bewirkt, dass das Dosisanzeigeelement drehfest mit dem Rotationsglied verbunden ist. Im unbetätigten Zustand der Vorrichtung sind das Rotationsglied und das Gehäuse im Bezug zueinander drehfest, insbesondere die erste und die zweite Kupplungsstruktur in einem Eingriff, wobei die dritte und zweite oder optional die dritte und vierte Kupplungsstruktur eingriffsfrei sind. Im betätigten Zustand sind die dritte und zweite, optional die dritte und vierte Kupplungsstruktur im Eingriff, wobei die erste und zweite Kupplungsstruktur vorzugsweise voneinander gelöst sind.

Besonders vorteilhaft ist es, wenn das Dosisanzeigeelement bereits drehfest mit dem Rotationsglied gekoppelt ist und das Rotationsglied noch drehfest mit dem Gehäuse verbunden ist, während das Betätigungsglied für die Betätigung in Bezug auf das Gehäuse verschoben wird. Hierdurch wird sichergestellt, dass das Dosisanzeigeelement zuerst sicher mit dem Rotationsglied gekoppelt ist, wenn das Rotationsglied für eine Drehung relativ zu dem Gehäuse freigegeben ist. Mit anderen Worten gibt es zwischen der betätigten und unbetätigten Position des Betätigungsglieds eine Zwischenposition, in der das Rotationsglied sowohl drehfest mit dem Gehäuse als auch drehfest mit dem Dosisanzeigeelement gekoppelt ist. Insbesondere können die erste und die zweite und die dritte und die zweite, optional die dritte und die vierte Kupplungsstruktur gleichzeitig in einem Eingriff sein, nämlich dann, wenn das Betätigungsglied seine Zwischenposition einnimmt.

In allgemein bevorzugten Ausführungen kann das Dosisanzeigeelement einen Anschlag, wie z. B. einen Nulldosisanschlag aufweisen, der von einem Gegenanschlag, insbesondere einem Nulldosisgegenanschlag wegbewegt wird, wenn eine Dosiserhöhung vorgenommen wird, und der zu dem Gegenanschlag hinbewegt wird, wenn eine Dosisverringerung vorgenommen wird, oder wenn die Vorrichtung für die Ausschüttung der eingestellten Produktdosis betätigt wird.

Insbesondere kann das Dosisanzeigeelement während des Einstellens der Produktdosis, d. h. bei Dosiserhöhung und Dosisverringerung, von dem Rotationsglied zumindest rotatorisch entkoppelt sein und bei der Betätigung der Vorrichtung zum Ausschütten der Produktdosis so mit dem Rotationsglied gekoppelt sein, dass eine Drehung des Rotationsglieds bewirkt, dass das Dosisanzeigeelement zu dem Gegenanschlag, d. h. der Nulldosisanschlag zu dem Nulldosisgegenanschlag hin bewegt wird. Sind der Nulldosisanschlag und der Nulldosisgegenanschlag in einem Anschlag oder einem Kontakt, wird hierdurch und insbesondere über die Kupplung, eine Drehung des Rotationsglieds und somit ein weiterer Vorschub des Vortriebsglieds relativ zu dem Gehäuse verhindert.

Zwischen dem Dosiseinstellglied und dem Dosisanzeigeelement kann eine Dosierkupplung vorgesehen sein, welche das Dosiseinstellglied mit dem Dosisanzeigeelement drehfest koppelt, wenn die Antriebs- und Dosiervorrichtung oder das Betätigungsglied unbetätigt ist, und rotatorisch entkoppelt, wenn die Antriebs- und Dosiervorrichtung oder das Betätigungsglied betätigt ist. Mit anderen Worten ausgedrückt, sind das Dosisanzeigeelement und das Dosierglied über die Dosierkupplung drehfest verbunden, wenn das Betätigungsglied unbetätigt ist, und ist das Dosisanzeigeelement relativ zu dem Dosiseinstellglied drehbar, wenn das Betätigungsglied betätigt ist. Durch Betätigung des Betätigungsglieds wird die Dosierkupplung geöffnet.

In vorteilhaften Weiterbildungen kann die Antriebs- und Dosiervorrichtung einen Mechanismus zum Verhindern des Einstellens einer Dosis, welche die Menge eines Medikaments in dem Produktbehälter übersteigt, aufweisen. Insbesondere kann dieser Mechanismus die Drehung des Dosierglieds in eine Richtung, welche eine Dosiserhöhung bewirken würde blockieren, insbesondere auch wenn der Maximaldosisanschlag des Dosisanzeigeelements und der Maximaldosisgegenanschlag noch nicht in einem Eingriff sind oder wenn in der Zeigeeinrichtung eine Dosis angezeigt wird, die kleiner ist als die maximal einstellbare Produktdosis. Der Mechanismus verhindert somit, dass eine Dosis eingestellt werden kann, welche die Restmenge des in dem Produktbehälters enthaltenen Produkts übersteigt, wodurch die Gefahr einer Fehlanwendung der Antriebs- und Dosiervorrichtung verringert wird. Der Mechanismus kann z. B. einen Begrenzer aufweisen, der zwischen zwei Teilen angebracht ist, von denen sich eines relativ zu dem anderen während der Dosiseinstellung dreht und bei der Betätigung, d. h. der Dosisausschüttung nicht dreht. Z. B. kann der Begrenzer zwischen dem Dosiseinstellglied, das insbesondere als Dosiseinstellknopf oder Dosiseinstellhülse ausgestaltet sein kann, und dem Gehäuse oder einem gehäusefesten Element angeordnet sein. Der Begrenzer, das Dosiseinstellglied und das Gehäuse können so miteinander gekoppelt sein, dass eine relative Drehung, insbesondere während der Dosiseinstellung, zwischen dem Dosiseinstellglied und dem Gehäuse bewirkt, dass sich der Begrenzer zu einer Stoppposition hinbewegt, in welcher der Begrenzer das Einstellen einer Dosis verhindert, die die Menge eines Produkts in dem Produktbehälter übersteigt. Beispiele für entsprechend geeignete Begrenzer werden in der WO 2010/149209 oder in der WO 01/19434 A1, insbesondere in deren Figur 3 offenbart. Beispielsweise kann der Begrenzer ein Innengewinde aufweisen, welches in dem Eingriff mit einem Außengewinde des Gehäuses ist. Insbesondere kann der Begrenzer an seiner Außenseite eine Längsführung aufweisen, mit der er in einem Eingriff mit dem Dosiseinstellglied ist, so dass das Dosiseinstellglied relativ zu dem Begrenzer drehfest ist. Alternativ kann das Gehäuse die Längsführung für den Begrenzer aufweisen, so dass der Begrenzer relativ zu dem Gehäuse drehfest ist und kann der Begrenzer ein Gewinde, insbesondere Außengewinde aufweisen, welches in einen Gewinde, insbesondere Innengewinde des Dosiseinstellglieds eingreift.

Die Stoppposition wird durch einen Anschlag für den Begrenzer definiert, wobei der Anschlag von dem Gehäuse oder dem Dosiseinstellglied oder einem zumindest axial oder in Umfangsrichtung gehäusefesten Mittel gebildet werden kann. Sind der Begrenzer und der Anschlag in einem Kontakt, ist eine Drehung des Dosiseinstellglieds in eine Drehrichtung, welche eine Erhöhung der Dosis bewirken würde, nicht mehr möglich oder blockiert.

In allgemein bevorzugten Weiterbildungen, insbesondere des ersten und zweiten Aspekts, kann die Antriebs- und Dosiervorrichtung mindestens einen Signalerzeugungsmechanismus aufweisen, der angepasst ist, während der Dosiseinstellung oder/und der Produktausschüttung ein akustisches und/oder taktiles Signal, insbesondere mechanisch, zu erzeugen. Ein solches Signal kann insbesondere als Klicksignal wahrgenommen werden. Zum Beispiel kann ein (erster) Signalerzeugungsmechanismus vorgesehen sein, der das Signal während der Dosiseinstellung erzeugt und optional als Dosiseinstellungssignalerzeugungsmechanismus bezeichnet werden kann. Ferner kann ein weiterer (zweiter) Signalerzeugungsmechanismus vorgesehen sein, der das Signal während der Produktausschüttung erzeugt und optional als Produktausschüttungssignalerzeugungsmechanismus bezeichnet werden kann. Alternativ kann ein (gemeinsamer) Signalerzeugungsmechanismus vorgesehen sein, der das Signal während der Dosiseinstellung und während der Produktausschüttung erzeugt.

Der Signalerzeugungsmechanismus kann allgemein zwischen zwei Teilen angeordnet sein, die sich bei der Dosiseinstellung oder/und der Produktausschüttung relativ zueinander bewegen, insbesondere drehen. Eines der Teile kann ein z.B. federnd angeordnetes Rastglied aufweisen, das in eine z.B. über den Umfang angeordnete Verzahnung des anderen der zwei Teile eingreift. Wird ein Teil relativ zu dem anderen verdreht, kann das Rastglied über die Verzahnung gleiten und dabei das Signal erzeugen. Die Verzahnung kann von einem Innenumfang oder einem Außenumfang oder einer Stirnfläche des Teils gebildet sein.

Der Signalerzeugungsmechanismus kann insbesondere zwischen Kupplungsglied und Lagerelement gebildet sein. Vorzugsweise drehen sich das Kupplungsglied und das Lagerelement während der Dosiseinstellung und der Produktausschüttung relativ zueinander, wodurch ein Signalerzeugungsmechanismus gebildet wird, der das Signal während der Dosiseinstellung und der Produktausschüttung erzeugt.

Der Signalerzeugungsmechanismus kann insbesondere zwischen Lagerelement und Rotationsglied gebildet sein, wobei das für das Lagerelement ausgeführte, zumindest in diesem Zusammenhang, auch für eine hierin beschriebene Schalthülse gilt. Vorzugsweise drehen sich das Lagerelement und das Rotationsglied, während, insbesondere nur während der Produktausschüttung relativ zueinander, wodurch ein Signalerzeugungsmechanismus gebildet wird, der das Signal während der Produktausschüttung erzeugt.

Der Signalerzeugungsmechanismus kann insbesondere zwischen Kupplungsglied und Rotationsglied gebildet sein. Vorzugsweise drehen sich das Kupplungsglied und das Rotationsglied, während, insbesondere nur während der Dosiseinstellung relativ zueinander, wodurch ein Signalerzeugungsmechanismus gebildet wird, der das Signal während der Dosiseinstellung erzeugt.
Die Erfindung wurde anhand mehrerer vorteilhafter Ausführungsformen beschrieben. Im Folgenden werden vorteilhafte Ausführungsformen anhand von Figuren beschrieben. Die dabei offenbarten Merkmale bilden den Gegenstand der Ansprüche, insbesondere der unabhängigen Ansprüche, insbesondere einzeln und in jeglicher Kombination mit den Ansprüchen oder/und der vorhergehenden Beschreibung vorteilhaft weiter. Es zeigen:
- Figur 1: eine Explosionsdarstellung der Einzelteile einer ersten Ausführungsform einer erfindungsgemäßen Antriebs- und Dosiervorrichtung,
- Figuren 2a-c: die Antriebs- und Dosiervorrichtung aus Figur 1 in einem Ausgangs- oder Auslieferungszustand, wobei Figur 2b eine Schnittansicht der Figur 2a ist und Figur 2c eine um 90° gedrehte Schnittansicht der Figur 2a entlang der Linie B-B ist,
- Figuren 3a-c: die Injektionsvorrichtung in den Ansichten aus Figur 2a in einem Zustand, in dem die maximal einstellbare Dosis eingestellt ist,
- Figuren 4a-c: die Ansichten aus Figuren 2a-c, jedoch in einem Zustand, bei dem die in den Figuren 3a-c eingestellte Dosis vollständig ausgeschüttet wurde und ein Betätigungsglied noch betätigt ist,
- Figuren 5a-c: die Ansichten aus den Figuren 2a-c, wobei ein Antriebsglied der Antriebs- und Dosiervorrichtung für eine Bewegung zur Dosiserhöhung gesperrt ist, weil die in dem Produktbehälter enthaltene Dosis geringer ist als die maximal einstellbare Dosis
- Figur 6: eine Explosionsdarstellung der Einzelteile einer zweiten Ausführungsform einer erfindungsgemäßen Antriebs- und Dosiervorrichtung,
- Figuren 7a-c: die Antriebs- und Dosiervorrichtung aus Figur 6 in einem Ausgangs- oder Auslieferungszustand, wobei Figur 7b eine Schnittansicht der Figur 7a ist und Figur 7c eine um 90° gedrehte Schnittansicht der Figur 7a entlang der Linie B-B ist,
- Figuren 8a-b: eine Explosionsdarstellung der Einzelteile einer dritten Ausführungsform einer erfindungsgemäßen Antriebs- und Dosiervorrichtung, wobei Figur 8b eine Schnittdarstellung der Figur 8a ist,
- Figuren 9a-c: die Antriebs- und Dosiervorrichtung aus den Figuren 8a und 8b in einem Ausgangs- oder Auslieferungszustand, wobei Figur 9b eine Schnittansicht der Figur 9a ist und Figur 9c eine um 90° gedrehte Schnittansicht der Figur 9a entlang der Linie B-B ist,
- Figuren 10a-b: eine Explosionsdarstellung der Einzelteile einer vierten Ausführungsform einer erfindungsgemäßen Antriebs- und Dosiervorrichtung, wobei Figur 10b eine Schnittdarstellung der Figur 10a ist,
- Figuren 11a-c: die Antriebs- und Dosiervorrichtung aus den Figuren 10a und 10b in einem Ausgangs- oder Auslieferungszustand, wobei Figur 11b eine Schnittansicht der Figur 11a ist und Figur 11c eine um 90° gedrehte Schnittansicht der Figur 11a entlang der Linie B-B ist,
- Figur 12: eine Explosionsdarstellung der Einzelteile einer fünften Ausführungsform einer erfindungsgemäßen Antriebs- und Dosiervorrichtung,
- Figuren 13a-c: die Antriebs- und Dosiervorrichtung aus Figur 12 in einem Ausgangs- oder Auslieferungszustand, wobei Figur 13b eine Schnittansicht der Figur 13a ist und Figur 13c eine um 90° gedrehte Schnittansicht der Figur 13a entlang der Linie B-B ist,
- Figur 14: eine Explosionsdarstellung der Einzelteile einer sechsten Ausführungsform einer erfindungsgemäßen Antriebs- und Dosiervorrichtung,
- Figuren 15a-c: die Antriebs- und Dosiervorrichtung aus Figur 14 in einem Ausgangs- oder Auslieferungszustand, wobei Figur 15b eine Schnittansicht der Figur 15a ist und Figur 15c eine um 90° gedrehte Schnittansicht der Figur 15a entlang der Linie B-B ist,
- Figur 16: eine modifizierte Anordnung von Ausschüttfedern und
- Figur 17: eine weitere modifizierte Anordnung von Ausschüttfedern.

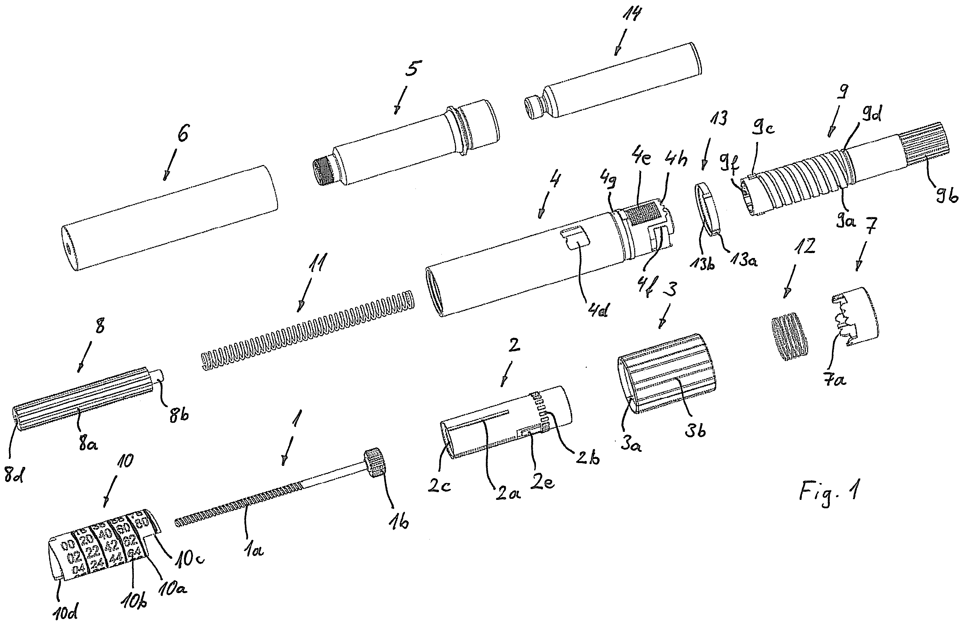

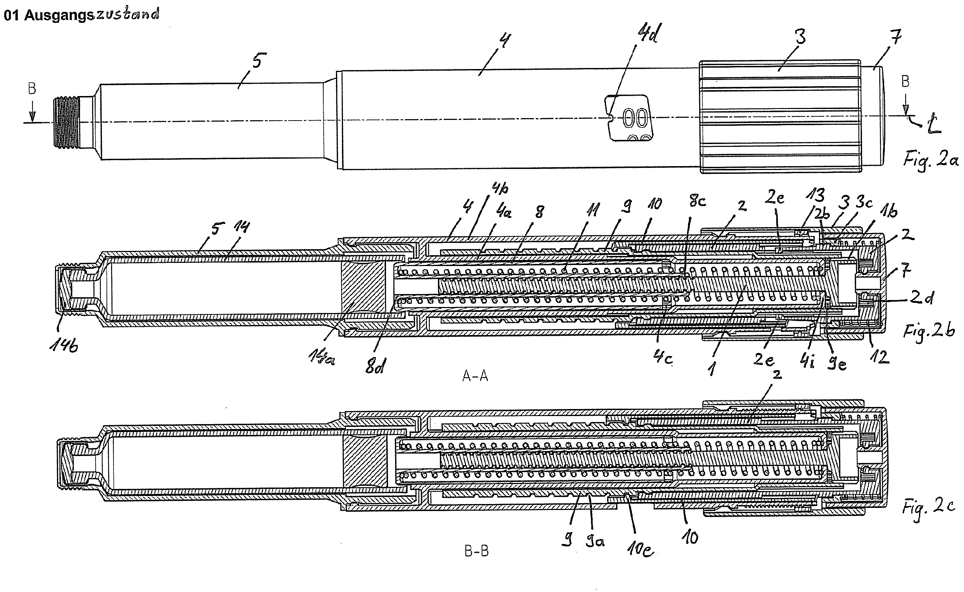

Die Antriebs- und Dosiervorrichtung umfasst in einer ersten Ausführungsform, wie z. B. aus den Figuren 1 und 2a bis 2c erkennbar ist, ein hülsenförmiges Gehäuse 4, welches eine Außenhülse 4b aufweist, welche vom Verwender mit einer Hand umgreifbar ist. Wie am besten aus Figur 2b erkennbar ist, umfasst das Gehäuse 4 ferner eine Innenhülse 4a, die ein Widerlager 4i bildet und konzentrisch zu der Außenhülse 4b angeordnet ist. Innenhülse 4a und Außenhülse 4b sind über einen Ringsteg miteinander verbunden. Zwischen der Außenhülse 4b und der Innenhülse 4a ist ein Ringspalt gebildet, in dem ein Dosisanzeigeelement 10, das insbesondere als Dosisanzeigetrommel, d. h. hülsenförmig ausgestaltet ist, ein Lagerelement 9 und ein Kupplungsglied 2, das hülsenförmig ist und insbesondere auch als Anzeigekupplung bezeichnet werden kann, angeordnet sind.

An dem distalen Ende des Gehäuses 4 ist eine hülsenförmige Produktbehälteraufnahme 5 aus einem vorzugsweise transparenten Material angeordnet, in der ein Produktbehälter 14 in der Gestalt einer Karpule aufgenommen ist. Der Produktbehälter 14 ist mittels der Produktbehälteraufnahme 5 mit dem Gehäuse 4 unlösbar verbunden, so dass die Antriebs- und Dosiervorrichtung insbesondere zusammen mit der Produktbehälteraufnahme 5 und dem Produktbehälter 14 eine Einweg-Injektionsvorrichtung bildet, die nach vollständiger Entleerung des Produktbehälters 14 als Ganzes entsorgt wird. Der Produktbehälter 14 weist an seinem distalen Ende ein Septum 14b auf, welches von einer durch am distalen Ende des Produktbehälters 14 bzw. der Produktbehälteraufnahme 5 anbringbaren Nadel durchstechbar ist. In dem Produktbehälter 14 ist ein Kolben 14a aufgenommen, wobei das auszuschüttende Produkt zwischen dem Septum 14b und dem Kolben 14a angeordnet ist. Eine Verschiebung des Kolbens 14a in Richtung Septum oder in distale Richtung, mithin Ausschüttrichtung, bewirkt eine Ausschüttung des in dem Produktbehälter 14 enthaltenen Produkts. In Figur 1 wird zusätzlich noch eine Schutzkappe 6 dargestellt, welche über der Produktbehälteraufnahme 5 anbringbar ist und vor dem Injizieren einer Dosis abgenommen wird.

Das Gehäuse 4, insbesondere die Innenhülse 4a ist in einem Eingriff mit einem hülsenförmigen Vortriebsglied 8, welches auch als Stößel bezeichnet werden kann. Das Vortriebsglied 8 ist relativ zu dem Gehäuse 4 drehfest und axial entlang der Längsachse L (Figur 2a) verschiebbar. Zwischen der Innenhülse 4a und dem Vortriebsglied 8 ist eine Führung mittels mindestens einer Längsrippe 8a und mindestens einer Längsführung 4c gebildet, welche eine Drehung des Vortriebsglieds 8 relativ zu dem Gehäuse 4 verhindert und eine Axialbewegung des Vortriebsglieds 8 relativ zu dem Gehäuse 4 erlaubt. Die Längsrippe 8a wird vorzugsweise von einer Außenhülse des Vortriebsglieds 8 gebildet. Das Vortriebsglied 8 weist eine Innenhülse 8b auf, welche in diesem Beispiel an ihrem proximalen Ende ein Innengewinde 8c aufweist, welches in ein Außengewinde 1a eines als Gewindestange ausgestalteten Rotationsglieds 1 eingreift. Das Vortriebsglied 8 ist so angeordnet, dass sein distales Ende 8d auf den Kolben 14a wirken, insbesondere gegen den Kolben 14a drücken kann.

Das Gehäuse 4, insbesondere das proximale Ende der Innenhülse 4a bildet das Widerlager 4i für eine Ausschüttfeder 11, die sich an dem Widerlager 4i und im Bereich des distalen Endes des Vortriebsglieds 8 abstützt. Die Feder 11 stützt sich mit ihrem distalen Ende an einem Ringsteg des Vortriebsglieds 8 ab, der die Außenhülse und die Innenhülse des Vortriebsglieds 8 verbindet. Die Feder 11 stützt sich mit ihrem proximalen Ende an dem von dem Gehäuse 4 gebildeten und nach innen ragenden Ringsteg ab, welcher das Widerlager 4i bildet.

Die Ausschüttfeder 11 ist als Schrauben- oder Wendelfeder gebildet, die als Druckfeder wirkt und danach strebt, das Widerlager 4i und das Vortriebsglied 8 auseinander zu drücken, d. h. das Vortriebsglied 8 in distale Richtung relativ zu dem Gehäuse 4 zu verschieben. Die Ausschüttfeder 11 ist bei der Auslieferung, d. h. im Ausgangszustand der Antriebs- und Dosiervorrichtung so stark vorgespannt, dass die in ihr gespeicherte Energie ausreicht, das in dem Produktbehälter 14 enthaltene Produkt im Wesentlichen vollständig auszuschütten, insbesondere mit mehreren Einzelausschüttungen, zwischen denen jeweils eine neue Dosiseinstellung vorgenommen wird. Der Vorteil an einer so stark vorgespannten Feder ist, dass die Feder 11 nicht z. B. während der Dosiseinstellung gespannt werden muss, wodurch für den Verwender der Vorrichtung eine Kraft sparende, d. h. einfachere Dosiseinstellung vornehmbar ist.

Der Gewindeeingriff zwischen dem Vortriebsglied 8 und dem Rotationsglied 1 ist so groß, dass keine Selbsthemmung des Gewindeeingriffs auftritt, d. h., dass das Rotationsglied 1 aufgrund der Axialkraft der Ausschüttfeder 11 relativ zu dem Vortriebsglied 8 um die Längsachse L drehbar oder rotierbar ist.

Das Rotationsglied 1 ist als Gewindestange ausgebildet, die das Außengewinde 1a bildet und an ihrem proximalen Ende einen vergrößerten Durchmesser, insbesondere in der Gestalt eines verbreiterten Kopfs aufweist. An dem Kopf sind parallel zur Längsachse L Zähne 1b angeordnet die als zweite Kupplungsstruktur dienen, wie weiter unten beschrieben wird. An dem Kopf ist eine ringförmige, vom Durchmesser her gegenüber dem Kopf verkleinerte Reibfläche angeordnet, die in einem Kontakt mit dem nach innen ragenden, das Widerlager 4i bildenden Ringsteg des Gehäuses 4 ist. Durch den verringerten Durchmesser der ringförmigen Reibfläche wird der Angriffspunkt der resultierenden Reibkraft näher zur Längsachse L hin verschoben, wodurch das Reibmoment zwischen Rotationsglied 1 und Gehäuse 4 herabgesetzt wird.

Durch Drehung des Rotationsglieds 1 relativ zu dem Gehäuse 4 und dem Vortriebsglied 8 kann die Feder 11 das Vortriebsglied 8 um einen Ausschütthub in distale Richtung verschieben, der proportional zu dem Drehwinkel des Rotationsglieds 1 ist. Durch wahlweises Sperren und Freigeben des Rotationsglieds 1, was durch Betätigen eines als Betätigungsknopf ausgestalteten Betätigungsglieds 7 bewirkt werden kann, kann die Bewegung des Vortriebsglieds 8 relativ zu dem Gehäuse 4, d. h. der Ausschütthub des Vortriebsglieds 8 auf eine vorteilhafte Weise gesteuert werden.

Die Antriebs- und Dosiervorrichtung weist ferner ein Lagerelement 9 auf, welches auch als Anzeigetrommellagerelement bezeichnet werden kann und relativ zu dem Gehäuse 4 drehfest aber entlang der Längsachse L verschiebbar angeordnet ist. Das Lagerelement 9 ist hülsenförmig und umgibt vorzugsweise die Innenhülse 4a des Gehäuses 4, wobei insbesondere die Außenhülse 4b das Lagerelement 9 umgibt. Das Lagerelement 9 ist in einem Eingriff mit dem Gehäuse 4, insbesondere der Innenhülse 4a, der eine Längsbewegung des Lagerelements 9 relativ zu dem Gehäuse 4 zulässt, aber eine Drehbewegung verhindert. Der Eingriff kann durch eine Längsführung 9f zwischen dem Lagerelement 9 und der Innenhülse 4a gebildet werden.

Das Lagerelement 9 weist ein Gewinde 9a, insbesondere ein Außengewinde auf, in welches ein Gewinde 10e, insbesondere ein Innengewinde, des Dosisanzeigeelements 10 eingreift. Durch diesen Gewindeeingriff ist das Anzeigeelement 10 relativ zu dem Lagerelement 9 schraubbar.

Die erste Ausführungsform umfasst ferner einen Signalerzeugungsmechanismus 2e, 9b, der ein akustisches und/oder taktiles Signal bei der Dosiseinstellung und der Produktausschüttung erzeugt. Der Signalerzeugungsmechanismus 2e, 9b ist zwischen dem Kupplungsglied 2 und dem Lagerelement 9 angeordnet und umfasst insbesondere ein Rastglied 2e und eine Verzahnung 9b. Das Lagerelement 9 weist eine sich über den Umfang, insbesondere Außenumfang erstreckende Verzahnung 9b auf. Das Kupplungsglied 2 weist das federnd angeordnete und in die Verzahnung 9b eingreifende Rastglied 2e auf.

Das Lagerelement 9 weist an seinem proximalen Ende die über seinen Umfang erstreckte Verzahnung 9b auf, die z.B. zur Einstellung von diskreten dosisproportionalen Winkelschritten und/oder zur Erzeugung eines leichten Widerstands bei der Dosiseinstellung und/oder zur Erzeugung des akustischen oder taktilen Signals, wie z. B. eines hör- und fühlbaren Klicks bei der Dosiseinstellung und der Produktausschüttung, dient. In die Verzahnung 9b greifen zwei Rastglieder 2e ein, welche federnd an Rastarmen angeordnet sind und von dem Kupplungsglied 2 gebildet werden. Das Kupplungsglied 2 ist axialfest mit dem Lagerelement 9 und relativ zu dem Lagerelement 9 drehbar verbunden. Hierzu greift das Kupplungsglied 2 mittels einer Ringnut 2c in eine über den Umfang des Lagerelements 9 sich erstreckende ringförmige Abragung 9d ein. Eine Drehung des hülsenförmigen Kupplungsglieds 2 relativ zu dem Lagerelement 9 bewirkt, dass die Rastglieder 2e über die Verzahnung 9b rasten und dabei das akustische und/oder taktile Signal erzeugen.

Das Dosisanzeigeelement 10 ist drehfest, aber axial verschiebbar mit dem Kupplungsglied 2 verbunden, insbesondere in einem Eingriff. Dieser Eingriff umfasst eine Längsführung 2a, welche bewirkt, dass das Dosisanzeigeelement 10 relativ zu dem Kupplungsglied 2 drehfest, aber axial verschiebbar ist. Eine Drehung des Kupplungsglieds 2 relativ zu dem Lagerelement 9 bewirkt aufgrund der drehfesten Verbindung zwischen Kupplungsglied 2 und Dosisanzeigeelement 10, dass das Dosisanzeigeelement 10 ebenfalls gedreht und aufgrund des Gewindeeingriffs in das Gewinde 9a an dem Lagerelement 9 entlang geschraubt wird, insbesondere zusätzlich zu dem aufgrund der Rastglieder 2e erzeugten Klickgeräusche.

Das Dosisanzeigeelement 10 weist über seinen Außenumfang eine sich entsprechend der Steigung des Gewindes 10e wendeiförmig ersteckende Dosisskala 10b auf, die eine Vielzahl nacheinander angeordnete Skalenwerte umfasst. In dem gezeigten Beispiel kann mit der Antriebs- und Dosiervorrichtung eine maximale Dosis von 80 I.U. eingestellt werden, wobei die Skala von 0 bis 80 reicht und die Dosiswerte in Zweierschritten angegeben sind.

Ebenfalls entsprechend der Steigung des Gewindes 10e ist eine Markierung 10a wendelförmig über den Außenumfang des Dosisanzeigeelements 10 angeordnet. Diese Markierung 10a dient - wie weiter unten beschrieben wird - zur Anzeige, ob die Vorrichtung betätigt oder unbetätigt ist. Die Markierung 10a ist eine optionale Einrichtung. Sie kann sich entlang der ganzen Dosisskala 10b oder nur Teilen oder einem einzelnen Skalenwert davon erstrecken. Insbesondere kann sie bei betätigter Antriebs- und Dosiervorrichtung nur gegen Ende der Produktausschüttung oder in der Nulldosisposition sichtbar sein.

Das Dosisanzeigeelement 10 weist an seinem z.B. proximalen Ende eine in Umfangsrichtung weisende und wirkende Anschlagfläche 10c auf, die als Nulldosisanschlag bezeichnet wird. Das Dosisanzeigeelement 10 weist an seinem, z.B. dem proximalen Ende entgegengesezten, distalen Ende eine in Umfangsrichtung weisende und wirkende Anschlagfläche 10d auf, welche als Maximaldosisanschlag bezeichnet wird.

Das Dosisanzeigelement 10 ist an dem Lagerelement 9 zwischen einer Nulldosisposition und einer Maximaldosisposition hin und her schraubbar. In der Nulldosisposition verhindert der Nulldosisanschlag 10c im Zusammenwirken mit einem von dem Gehäuse 4 gebildeten Nulldosisgegenanschlag 4f die Drehung des Dosisanzeigeelements 10 in eine erste Drehrichtung, nämlich eine Drehrichtung, die bewirken würde, dass eine kleinere Dosis als Null eingestellt wird. In dieser Nulldosisposition ist das Dosisanzeigeelement 10 in die entgegengesetzte, d. h. zweite Drehrichtung drehbar.

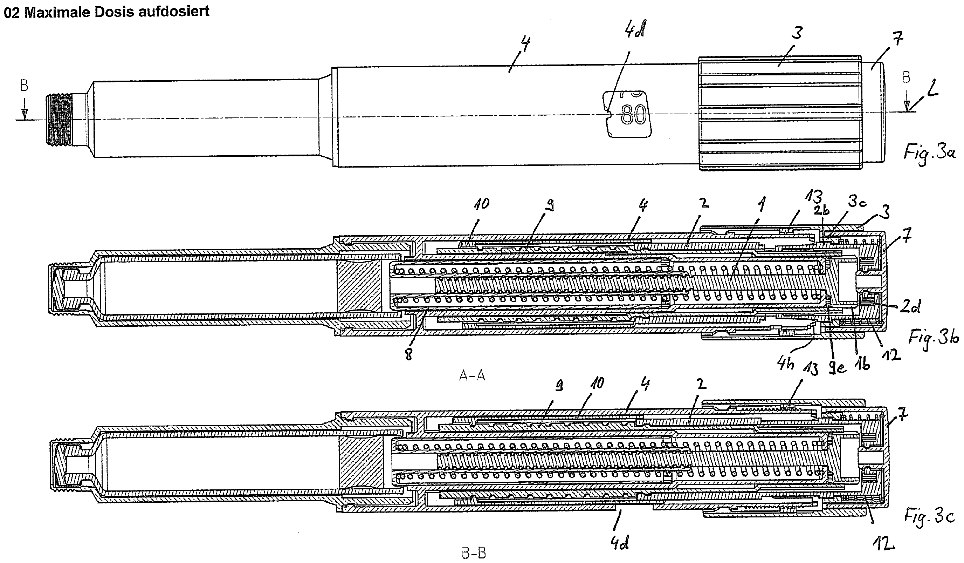

In der Maximaldosisposition, die z. B. in Figur 3a gezeigt wird, verhindert der Maximaldosisanschlag 10d im Zusammenwirken mit einem Maximaldosisgegenanschlag 9c, der von dem Lagerelement 9 gebildet wird, die Drehung des Dosisanzeigeelements 10 in die zweite Drehrichtung, welche eine Erhöhung der Dosis über den maximal einstellbaren Wert hinaus bewirken würde. Die Drehung in die erste Drehrichtung ist in der Maximaldosisposition möglich. Obgleich der Maximaldosisgegenanschlag 9c von dem Lagerelement 9 gebildet wird, kann abweichend von diesem Beispiel der Maximaldosisgegenanschlag 9c optional von dem Gehäuse 4 gebildet werden. Der Nulldosisgegenanschlag kann abweichend von dem gezeigten Beispiel von dem Lagerelement 9 gebildet werden.

Das Gehäuse 4 weist eine Zeigeeinrichtung 4d in der Gestalt eines Fensters auf, welches den Blick auf die Skala 10b des Dosisanzeigeelements 10 frei gibt. An dem Gehäuse 4 ist ein Dosierglied 3 in der Gestalt eines Dosierknopfs drehbar aber axial fest gelagert. Hierfür weist das Gehäuse 4 eine Ringnut 4g auf, in welche insbesondere eine Ringschulter des Dosierglieds 3 eingreift. Das Dosierglied 3 weist über seinen Außenumfang eine Griffstruktur 3b auf, welche es dem Verwender der Vorrichtung erleichtert, das Dosierglied 3 relativ zu dem Gehäuse 4 zu verdrehen. Im unbetätigten Zustand der Vorrichtung bewirkt eine Drehung des Dosierglieds 3 eine Drehung oder Schraubbewegung des Dosisanzeigeelements 10, wodurch die gewünschte Dosis einstellbar und in der Zeigeeinrichtung 4d ablesbar ist.

An dem Dosierglied 3 ist ein Betätigungsglied 7 in der Gestalt eines Betätigungsknopfs angeordnet, welches relativ zu dem Dosierglied 3 für eine Betätigung der Vorrichtung zur Produktausschüttung bewegbar ist, insbesondere entlang der Längsachse L. Das Betätigungsglied 7 bildet das proximale Ende der Vorrichtung und ist vom Daumen der Hand des Verwenders, welche das Gehäuse 4 umgreift, auf einfache Weise betätigbar, insbesondere relativ zu dem Gehäuse 4 und/oder zu dem Dosierglied 3 verschiebbar. Das Kupplungsglied 2 ist relativ zu dem Betätigungsglied 7, insbesondere bei gelöster Dosierkupplung 2b, 3c, drehbar und axial fest. Vorzugsweise ist das Betätigungsglied 7 mit dem Kupplungsglied 2 axial fest aber drehbar verschnappt.

Die Antriebs- und Dosiervorrichtung weist ferner eine Rücksetz- oder Kupplungsfeder 12 auf, die beim Betätigen, insbesondere Drücken des Betätigungsglieds 7 gespannt wird und die das Lagerelement 9 und/oder das Betätigungsglied 7 in seine unbetätigte Position zurücksetzt, wenn das Betätigungsglied 7 unbetätigt ist. Eine Betätigung des Betätigungsglieds 7 bewirkt neben seiner axialen Verschiebung ebenfalls die axiale Verschiebung des Lagerelements 9 entlang der Längsachse L. Die Feder 12 stützt sich mit ihrem distalen Ende vorzugsweise an dem Dosierglied 3 und mit ihrem proximalen Ende vorzugsweise an dem Betätigungsglied 7 ab. Die Feder 12 ist vorzugsweise eine Schrauben- oder Wendelfeder, welche als Druckfeder wirkt.

Das Dosierglied 3 ist relativ zu dem Betätigungsglied 7 drehfest. Das Betätigungsglied 7 durchgreift eine nach innen ragende Schulter des Dosierglieds 3. An dem distalen Ende des vorzugsweise topfförmig ausgestalteten Betätigungsglieds 7 sind mehrere Zähne gebildet, die zusammen eine Verzahnung 7a bilden, welche durch Betätigung des Betätigungsglieds 7 mit einer an dem Gehäuse 4 gebildeten Verzahnung 4h, insbesondere am proximalen Ende des Gehäuses 4, in einen Eingriff geraten, wodurch das Dosierglied 3 in Bezug auf das Gehäuse 4 drehfest ist. Dies bewirkt, dass bei betätigter Vorrichtung eine Dosiseinstellung, d. h. eine Verdrehung des Dosierglieds 3 relativ zu dem Gehäuse 4 nicht möglich ist, sondern erst dann, wenn das Betätigungsglied 7 unbetätigt ist.

Das Dosierglied 3 bildet eine Kupplungsstruktur 3c, insbesondere an der nach innen ragenden Schulter. Die Kupplungsstruktur 3c wirkt bei unbetätigtem Betätigungsglied 7 mit einer Kupplungsstruktur 2b am äußeren Umfang des Kupplungsglieds 2 zusammen. Im unbetätigten Zustand des Betätigungsglieds 7 sind das Dosierglied 3 und das Kupplungsglied 2 relativ zueinander drehfest aufgrund dieses Kupplungseingriffs. Die Kupplung zwischen Dosierglied 3 und Kupplungsglied 2 kann auch als Dosierkupplung 2b, 3c bezeichnet werden, die bei der Dosiseinstellung, d. h. bei unbetätigtem Betätigungsglied 7 eingerückt und bei der Dosisausschüttung, d. h. bei betätigtem Betätigungsglied 7 ausgerückt ist, wobei die Kupplung im eingerückten Zustand Drehmoment überträgt und im ausgerückten Zustand kein Drehmoment überträgt. Die Dosierkupplung 2b, 3c wird durch eine Verschiebung des Kupplungsglieds 2 relativ zu dem Gehäuse 4 ausgerückt oder geöffnet, insbesondere aufgrund der Betätigung des Betätigungsglieds 7.

Das Lagerelement 9 weist an seinem proximalen Ende am Innenumfang eine erste Kupplungsstruktur 9e auf, welche durch über den Umfang angeordnete Klauen oder Zähne gebildet werden, die mit den die zweite Kupplungsstruktur 1b bildenden Zähnen oder Klauen des Rotationsglieds 1 in einem Eingriff sind, insbesondere bei unbetätigtem Betätigungsglied 7. Durch diesen Kupplungseingriff ist das Rotationsglied 1 in Bezug auf das Gehäuse 4 drehfest. Das Kupplungsglied 2 weist ferner eine dritte Kupplungsstruktur 2d an einem Innenumfang auf, die mehrere über den Umfang verteilte Zähne oder Klauen aufweist. Die dritte Kupplungsstruktur 2d ist so angeordnet, dass sie bei Betätigung des Betätigungsglieds 7 in einen drehfesten Eingriff mit dem Rotationsglied 1 gelangt, insbesondere mit der zweiten Kupplungsstruktur 1b oder alternativ einer von der zweiten Kupplungsstruktur 1b separaten, in diesem Beispiel jedoch nicht gezeigten, vierten Kupplungsstruktur.

Während das Betätigungsglied 7 für die Betätigung relativ zu dem Dosierglied 3 entlang der Längsachse L verschoben wird, gerät zunächst die dritte Kupplungsstruktur 2d in einen Eingriff mit der zweiten Kupplungsstruktur 1b. Durch weiteres Verschieben des Betätigungsglieds 7 relativ zu dem Dosierglied 3 gerät die erste Kupplungsstruktur 9e aus dem Eingriff mit der zweiten Kupplungsstruktur 1b. Bevor, nachdem oder gleichzeitig mit dem Lösen des Eingriffs der ersten Kupplungsstruktur 9e mit der zweiten Kupplungsstruktur 1b gerät die Kupplungsstruktur 2b mit der Kupplungsstruktur 3c außer Eingriff und/oder die Verzahnung 7a mit der Verzahnung 4h in einen Eingriff.

Insbesondere dadurch, dass die erste Kupplungsstruktur 9e von der zweiten Kupplungsstruktur 1b gelöst ist, kann die Ausschüttfeder 11 sich entspannen, wobei das Rotationsglied 1 relativ zu dem Gehäuse 4 verdreht wird und aufgrund des Eingriffs der zweiten Kupplungsstruktur 1b mit der dritten Kupplungsstruktur 2d das Kupplungsglied 2 und somit auch das Dosisanzeigeelement 10 relativ zu dem Gehäuse 4 verdreht werden, wodurch das Dosisanzeigeelement 10 in seine Nulldosisposition zurück geschraubt und das Vortriebsglied 8 proportional zu dem sich insbesondere in Umfangsrichtung erstreckenden Abstand zwischen Nulldosisanschlag 10c und Nulldosisgegenanschlag 4f um einen Ausschütthub relativ zu dem Gehäuse 4 in distale Richtung verschoben wird. Die Drehung des Kupplungsglieds 2 relativ zu dem Lagerelement 9 bewirkt, dass die Rastglieder 2e über die Verzahnung 9b insbesondere in dosisproportionalen Winkelschritten rasten und dabei das akustische und /oder taktile Signal erzeugen.

Die Antriebs- und Dosiervorrichtung weist einen Dosisbegrenzer 13 in der Gestalt eines Rings, eines Ringsegments oder einer Mutter auf, der an seinem Innenumfang ein Gewinde 13b aufweist, das in ein an einem Außenumfang des Gehäuses 4 angeordnetes Gewinde 4e eingreift, so dass der Begrenzer 13 relativ zu dem Gehäuse 4 schraubbar ist. Am Außenumfang weist der Begrenzer 13 ein Eingriffsglied 13a auf, welches in eine Längsführung 3a am Innenumfang des Dosierglieds 3 eingreift, so dass das der Dosisbegrenzer 13 relativ zu dem Dosierglied 3 drehfest aber axial verschiebbar ist. An dem Dosierglied 3 oder dem Gehäuse 4 ist ein Stoppanschlag gebildet, von dem der Begrenzer 13 proportional zu der maximal aus dem Produktbehälter 14 ausschüttbaren Produktmenge beabstandet ist. Da bei der Dosiseinstellung das Dosierglied 3 relativ zu dem Gehäuse 4 verdreht und bei einer Dosisausschüttung nicht verdreht wird, kann durch den Begrenzer 13 ein Zählwerk gebildet werden, welches die bereits ausgeschütteten Einzeldosen und die aktuell eingestellte Dosis addiert und sich dementsprechend immer näher an den Stoppanschlag des Dosierglieds 3 oder des Gehäuses 4 annähert. Eine Dosiserhöhung bewirkt, dass der Begrenzer 13 zu dem Stoppanschlag hin bewegt wird. Eine Dosisverringerung bewirkt, dass der Begrenzer 13 von dem Stoppanschlag weg bewegt wird. Ist die in dem Produktbehälter 14 angegebene Restdosis geringer als die maximal mit der Antriebs- und Dosiervorrichtung einstellbare Dosis, gerät der Begrenzer 13 in einen Kontakt mit dem Stoppanschlag, so dass eine Verdrehung des Dosierglieds 3 relativ zu dem Gehäuse 4 in eine Drehrichtung, die eine Erhöhung der Dosis zur Folge hätte, blockiert wird.

Die aus der ersten, zweiten und dritten sowie optional der vierten Kupplungsstruktur 9e, 1b, 2d gebildete Kupplung kann aufgrund ihres Zusammenspiels auch als Ausschüttkupplung bezeichnet werden.

In den Figuren 2a bis 2c wird die Antriebs- und Dosiervorrichtung, die auch als Injektionsvorrichtung bezeichnet werden kann, in ihrem Ausgangs- oder Auslieferungszustand, insbesondere vor einer ersten Verwendung gezeigt. Die in der Zeigeeinrichtung 4d angezeigte Produktdosis ist 0. Ein Betätigen des Betätigungsglieds 7 hätte zur Folge, dass keine Dosis ausgeschüttet wird. Der Begrenzer 13 weist einen Abstand zu dem Stoppanschlag auf, welcher proportional zu der in dem Produktbehälter 14 enthaltenen oder ausschüttbaren Produktmenge ist, wie z. B. 300 I.U..

Zur Einstellung der Produktdosis wird das Dosiseinstellglied 3 relativ zu dem Gehäuse 4 verdreht, wodurch aufgrund des Kupplungseingriffs 2b, 3c das Kupplungsglied 2 und somit auch das Dosisanzeigeelement 10 relativ zu dem Gehäuse 4 verdreht werden. Dabei schraubt sich das Dosisanzeigeelement 10 entlang dem Lagerelement 9 aufgrund des Gewindeeingriffs des Gewindes 10e mit dem Gewinde 9a. Insbesondere wird der Abstand zwischen dem Nulldosisanschlag 10c und dem Nulldosisgegenanschlag 4f proportional zu der in der Zeigeeinrichtung 4d gezeigten Dosis erhöht. Außerdem wird bei der Drehung aufgrund des Überrastens der Rastglieder 2e über die Verzahnung 9b ein hörbares und fühlbares Signal erzeugt.

In den Figuren 3a bis 3c wird die Antriebs- und Dosiervorrichtung in einem Zustand gezeigt, in der eine maximal einstellbare Dosis eingestellt wurde, nämlich in diesem Beispiel 80 I.U., die in der Zeigeeinrichtung 4d ablesbar sind. Eine weitere Dosiserhöhung ist aufgrund des Zusammenwirkens, insbesondere des Kontakts des Maximaldosisanschlags 10d mit dem Maximaldosisgegenanschlag 9c nicht möglich. Der Dosisbegrenzer 13 ist, wie am besten aus den Figuren 3b und 3c erkennbar ist, entsprechend 80 I.U. weit an den Stoppanschlag herangerückt oder verschoben.

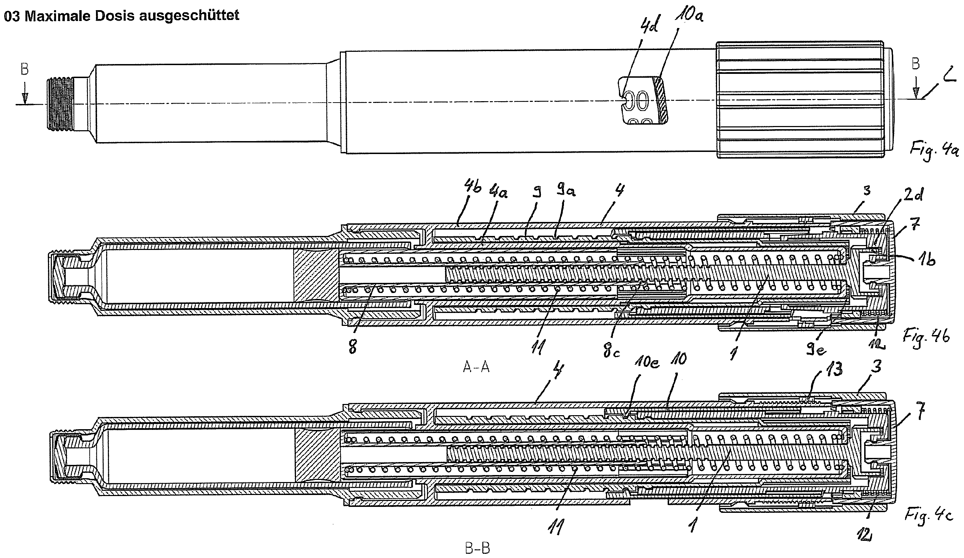

Zur Ausschüttung der z. B. in Figur 3a angezeigten Dosis wird das Betätigungsglied 7 betätigt, insbesondere gedrückt, d. h. relativ zu dem Gehäuse 4 und dem Dosierglied 3 in distale Richtung verschoben, wodurch das Kupplungsglied 2 und das Lagerelement 9 sowie das Dosisanzeigeelement 10 relativ zu dem Gehäuse 4 in distale Richtung verschoben werden, insbesondere gegen die Kraft der Kupplungs- oder Rücksetzfeder 12. Dadurch, dass das Dosisanzeigeelement 10 relativ zu dem Gehäuse 4 und der Zeigeeinrichtung 4d axial verschoben wird, erscheint die in Figur 1 gezeigte Markierung 10a in der Zeigeeinrichtung 4d (Figur 4a), wodurch der Verwender optisch ablesen kann, dass die Vorrichtung betätigt ist. Durch die Verschiebung des Dosisanzeigeelements 10 relativ zu dem Gehäuse 4 und der Zeigeeinrichtung 4d wird die Markierung 10a aus einer Position, in der sie von dem Gehäuse 4 verdeckt wird, in eine Position, in der sie in der Zeigeeinrichtung 14d gezeigt wird, entlang der Längsachse L verschoben.

Die Betätigung des Betätigungsglieds 7 bewirkt ferner, dass die dritte Kupplungsstruktur 2d mit der zweiten Kupplungsstruktur 1b einrückt und die erste Kupplungsstruktur 9e von der zweiten Kupplungsstruktur 1b ausrückt, so dass das Rotationsglied 1 in Bezug auf das Gehäuse 4 nicht mehr drehfest sondern drehbar ist und in Bezug auf das Kupplungsglied 2 und das Dosisanzeigeelement 10 drehfest ist. Die Betätigung des Betätigungsglieds 7 bewirkt ferner, dass die Dosierkupplung 2b, 3c ausrückt bzw. geöffnet wird und die Stirnverzahnung 7a in den Eingriff mit der Stirnverzahnung 4h gerät. Im betätigten Zustand des Betätigungsglieds 7 ist das Rotationsglied 1 relativ zu dem Dosisanzeigeelement 10 drehfest, wodurch sich das Rotationsglieds 1 und das Dosisanzeigeelement 10 gemeinsam relativ zu dem Gehäuse 4 drehen können. Durch die Kraft der in der Ausschüttfeder 11 gespeicherten Energie auf das Vortriebsglied 8 wird aufgrund des Gewindeeingriffs des Vortriebsglieds 8 mit dem Rotationsglied 1 eine Drehung des Rotationsglieds 1 und des Dosisanzeigeelements 10 relativ zu dem Gehäuse 4 bewirkt, wodurch sich das Dosisanzeigeelement 10 an dem Lagerelement 9 in Richtung Nulldosisposition zurückschraubt und wobei die in der Zeigeeinrichtung 14d angezeigte Dosis runterzählt. Gleichzeitig wird das Vortriebsglied 8 von der Ausschüttfeder 11 relativ zu dem Gehäuse 4 in distale Richtung um den Ausschütthub bewegt, welcher proportional zu der zuvor eingestellten Dosis ist. Wenn das Dosisanzeigeelement 10 seine Nulldosisposition erreicht hat (Figuren 4a bis 4c) ist die zuvor eingestellte Dosis oder Einzeldosis ausgeschüttet. Lässt der Verwender das in den Figuren 4a bis 4c noch als gedrückt dargestellte Betätigungsglied 7 los, setzt die Kupplungs- oder Rücksetzfeder 12 das Betätigungsglied 7, das Kupplungsglied 2, das Lagerelement 9 und das Dosisanzeigeelement 10 in die z. B. in Figur 2a gezeigte Position zurück, wobei die Markierung 10a wieder unter dem Gehäuse 4 verschwindet oder von dem Gehäuse 4 verdeckt wird. Beim Rücksetzen werden die genannten Elemente relativ zu dem Gehäuse 4 oder dem Dosierglied 3 in proximale Richtung verschoben.

Beim Zurücksetzen der Vorrichtung mittels der Feder 12 wird die erste Kupplungsstruktur 9e mit der zweiten Kupplungsstruktur 1b eingerückt und die dritte Kupplungsstruktur 2d von der zweiten Kupplungsstruktur 1b ausgerückt. Das Rotationsglied 1 ist nun wieder drehfest in Bezug auf das Gehäuse 4, wobei das Dosierglied 3 zusammen mit dem Dosisanzeigeelement 10 relativ zu dem Gehäuse 4 und/oder der Zeigeeinrichtung 4d und/oder dem Rotationsglied 1 für eine neue Einstellung einer Produktdosis oder Einzeldosis drehbar ist. Beim Zurücksetzen werden ferner die Stirnverzahnungen 7a und 4h aus dem Eingriff gelöst sowie die Dosierkupplung 2b, 3c wieder eingerückt, wodurch das Dosierglied 3 relativ zu dem Kupplungsglied 2 und dem Dosisanzeigeelement 10 drehfest ist.

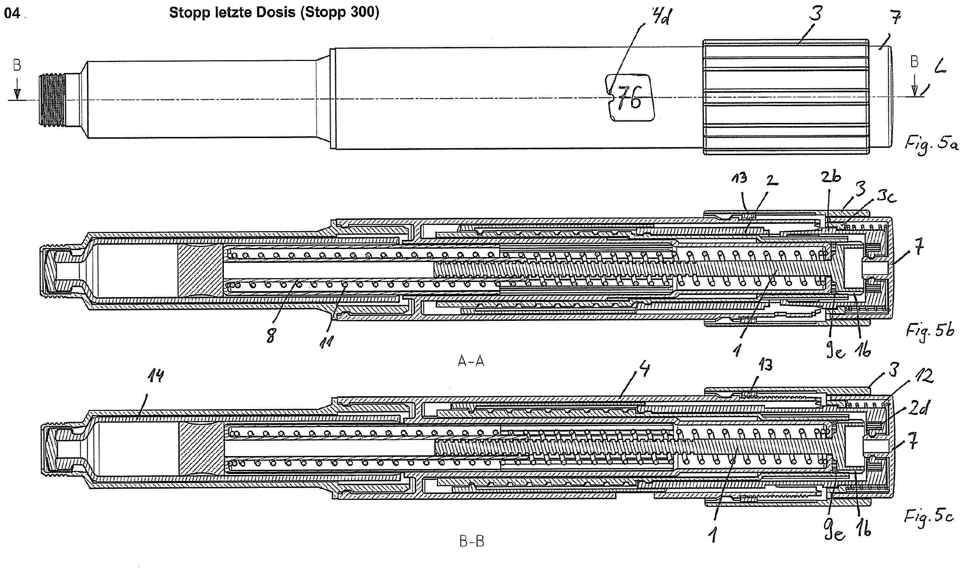

In Figur 5a wird die Antriebs- und Dosiervorrichtung in einer Position gezeigt, in welcher der Begrenzer 13 seine Stoppposition einnimmt, d. h. an dem Stoppanschlag anschlägt, wodurch der Begrenzer 13 eine Dosiseinstellung auf einen Wert, welcher die in dem Produktbehälter 14 enthaltene Restmenge übersteigt, blockiert. In dem gezeigten Beispiel sind noch 76 I.U. in dem Produktbehälter 14 enthalten, wobei mit der Antriebs- und Dosiervorrichtung maximal 80 I.U. einstellbar wären. Da der Begrenzer 13 bereits bei 76 I.U. in einem Kontakt mit dem Stoppanschlag ist, wird das Dosierglied 3 für eine Drehung in die zweite Richtung, welche eine Erhöhung der Dosis bewirken würde, gesperrt. Die Verringerung der Dosis ist jedoch durch Drehen des Dosierglieds 3 in die erste Drehrichtung möglich.

Durch Betätigen des Betätigungsglieds 7 wird die in der Zeigeeinrichtung 4d gezeigte Dosis ausgeschüttet. Da anschließend der Produktbehälter 14 vollständig entleert ist, wird die Antriebs- und Dosiervorrichtung oder Injektionsvorrichtung als Ganzes entsorgt. Es handelt sich somit um eine Einweg-Injektionsvorrichtung. Grundsätzlich können die hierin gezeigten Antriebs- und Dosiervorrichtungen aber auch in Verbindung mit Mehrweginjektionsvorrichtungen Anwendung finden, bei denen ein entleerter Produktbehälter 14 gegen einen neuen ausgetauscht wird.

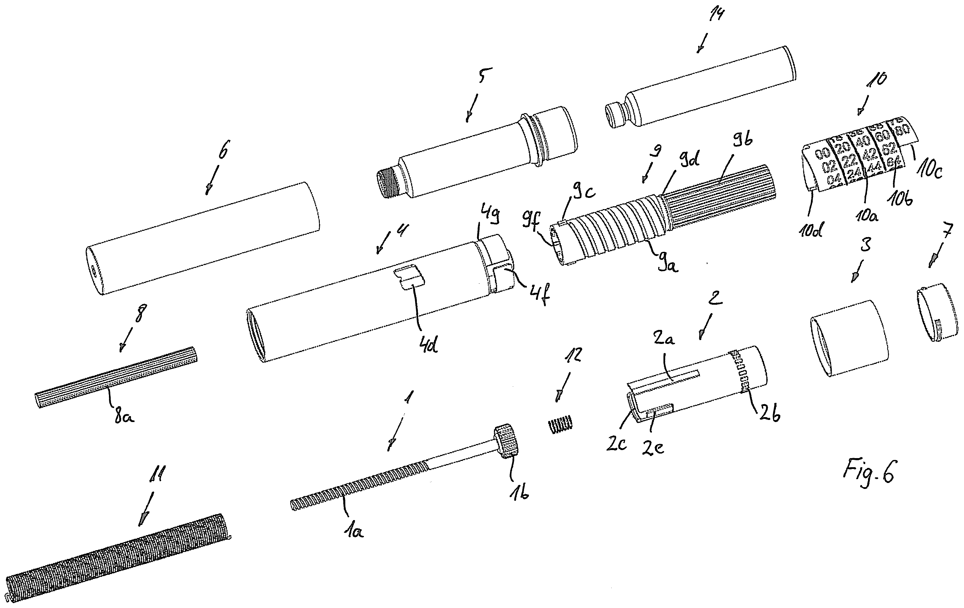

Eine zweite Ausführungsform einer Antriebs - und Dosiervorrichtung wird in den Figuren 6 bis 7c dargestellt. Im Folgenden werden die Merkmale beschrieben, mit denen sich diese Ausführungsform von der ersten unterscheidet, so dass im Übrigen auf die Beschreibung zu den Figuren 1 bis 5 verwiesen wird. Gleiche Bezugszeichen bezeichnen zumindest funktionell gleichartige Teile.

Die Antriebs- und Dosiervorrichtung unterscheidet sich insbesondere dadurch von der Ausführungsform aus den Figuren 1 bis 5, dass die Ausschüttfeder 11 eine Torsionsfeder ist.

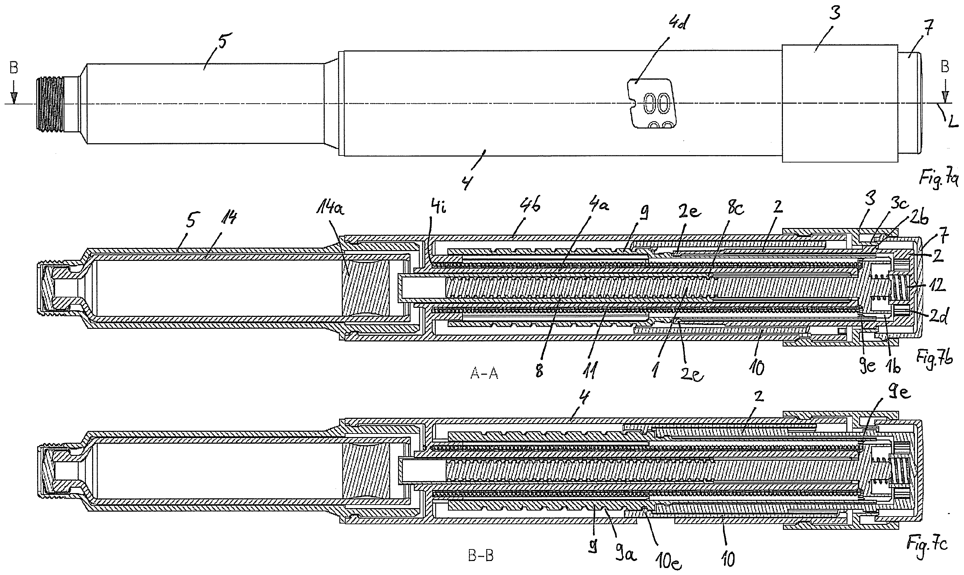

Wie am besten aus den Figuren 7b und 7c erkennbar ist, umfasst das Gehäuse 4 eine Innenhülse 4a, die konzentrisch zu der Außenhülse 4b angeordnet ist. Innenhülse 4a und Außenhülse 4b sind über einen Ringsteg miteinander verbunden. Das Gehäuse 4, insbesondere der Ringsteg bildet ein Widerlager 4i für das distale Ende der Feder 11.

Das Vortriebsglied 8 kommt z.B. ohne Innen- und Außenhülse aus. Die Längsrippe 8a wird vorzugsweise von dem hülsenförmigen Vortriebsglied 8 gebildet. Das Vortriebsglied 8 weist an seinem proximalen Ende ein Innengewinde 8c auf, welches in das Außengewinde 1a des als Gewindestange ausgestalteten Rotationsglieds 1 eingreift.

Das Gehäuse 4, insbesondere der die Außen- und Innenhülse 4a, 4b verbindende Ringsteg bildet das Widerlager 4i die Ausschüttfeder 11, die sich mit ihrem proximalen Ende an dem Widerlager 4i und im Bereich des Kopfs des Rotationsglieds 1 jeweils drehfest abstützt.

Die Ausschüttfeder 11 ist als Schrauben- oder Wendelfeder gebildet, die als Torsionsfeder wirkt und danach strebt, das Rotationsglied 1 relativ zu dem Gehäuse 4 zu verdrehen und dadurch mittelbar das Vortriebsglied 8 in distale Richtung relativ zu dem Gehäuse 4 zu verschieben. Die Ausschüttfeder 11 ist bei der Auslieferung, d. h. im Ausgangszustand der Antriebs- und Dosiervorrichtung so stark rotatorisch vorgespannt, dass die in ihr gespeicherte Energie ausreicht, das in dem Produktbehälter 14 enthaltene Produkt im Wesentlichen vollständig auszuschütten, insbesondere mit mehreren Einzelausschüttungen, zwischen denen jeweils eine neue Dosiseinstellung vorgenommen wird.

Der Gewindeeingriff zwischen dem Vortriebsglied 8 und dem Rotationsglied 1 kann, aber muss in diesem Beispiel nicht zwangsläufig so groß sein, dass keine Selbsthemmung des Gewindeeingriffs auftritt. Das Rotationsglied 1 ist aufgrund des Torsionsmoments der Ausschüttfeder 11 relativ zu dem Vortriebsglied 8 um die Längsachse L drehbar oder rotierbar.

Die Feder 12 stützt sich in diesem Beispiel mit ihrem distalen Ende vorzugsweise an dem Rotationsglied 1 und mit ihrem proximalen Ende vorzugsweise an dem Betätigungsglied 7 ab. Die Feder 12 ist vorzugsweise eine Schrauben- oder Wendelfeder, welche als Druckfeder wirkt.

Das Dosierglied 3 ist in einem drehfesten Eingriff mit dem Betätigungsglied 7. Das Betätigungsglied 7 ist relativ zu dem Dosierglied entlang der Längsachse L in Ausschüttrichtung verschiebbar. Obwohl nicht so dargestellt, könnte das Betätigungsglied 7 optional aber auch nach einem der anderen Beispiele ausgestaltet sein.

Das Rastglied 2e ist in dem gezeigten Beispiel im proximalen Bereich des Kupplungsglieds 2 angeordnet, abweichend von dem Beispiel aus den Figuren 1 bis 5, wo das Rastglied 2e in etwa mittig angeordnet ist. Die Drehung des Kupplungsglieds 2 relativ zu dem Lagerelement 9, wie z.B. bei der Produktausschüttung und bei der Dosiseinstellung, bewirkt, dass die Rastglieder 2e über die Verzahnung 9b insbesondere in dosisproportionalen Winkelschritten rasten und dabei das akustische und /oder taktile Signal erzeugen.

Durch das Drehmoment der in der Ausschüttfeder 11 gespeicherten Energie auf das Rotationsglied 1 wird eine Drehung des Rotationsglieds 1 und des Dosisanzeigeelements 10 relativ zu dem Gehäuse 4 bewirkt, wodurch sich das Dosisanzeigeelement 10 an dem Lagerelement 9 in Richtung Nulldosisposition zurückschraubt und wobei die in der Zeigeeinrichtung 14d angezeigte Dosis runterzählt. Gleichzeitig wird das Vortriebsglied 8 von der Ausschüttfeder 11 relativ zu dem Gehäuse 4 in distale Richtung um den Ausschütthub bewegt, welcher proportional zu der zuvor eingestellten Dosis ist. Wenn das Dosisanzeigeelement 10 seine Nulldosisposition erreicht hat, ist die zuvor eingestellte Dosis oder Einzeldosis ausgeschüttet.

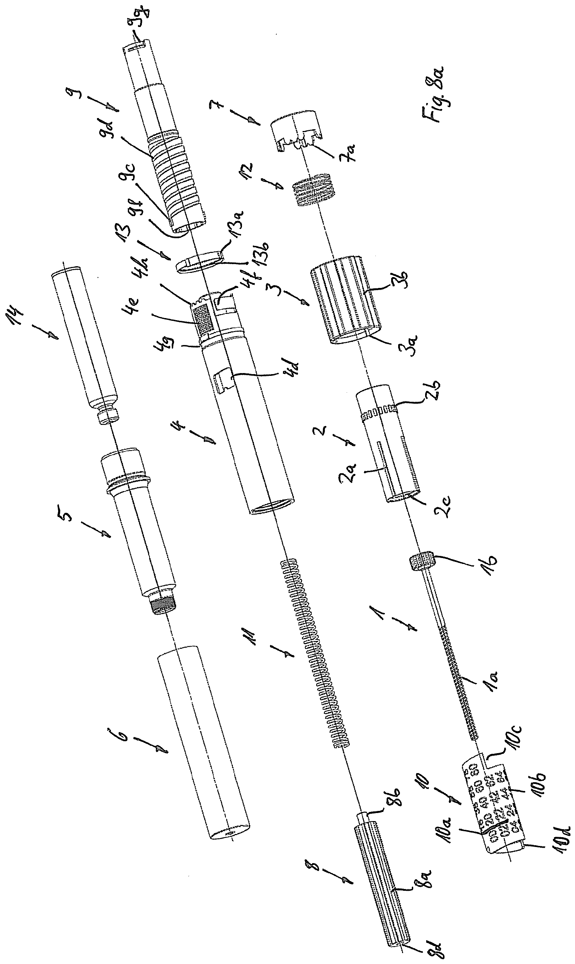

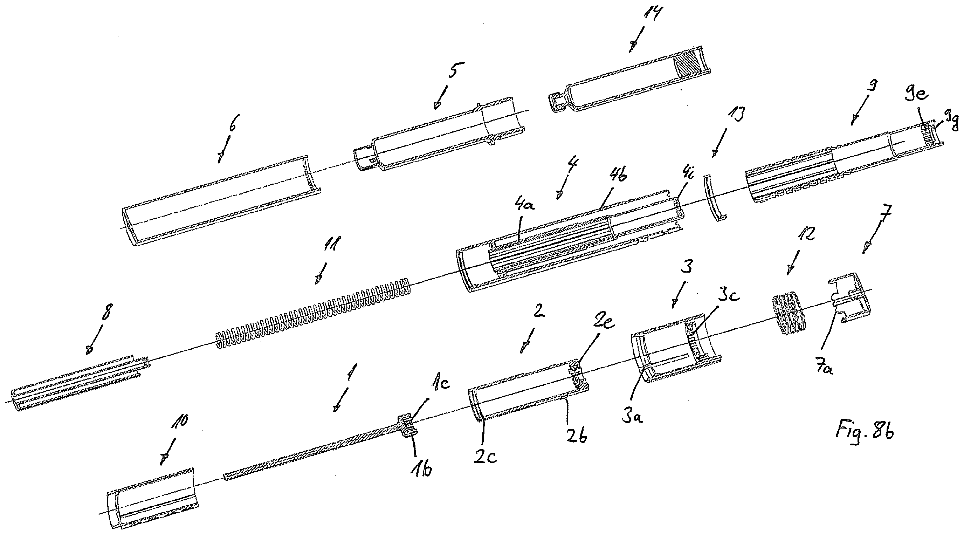

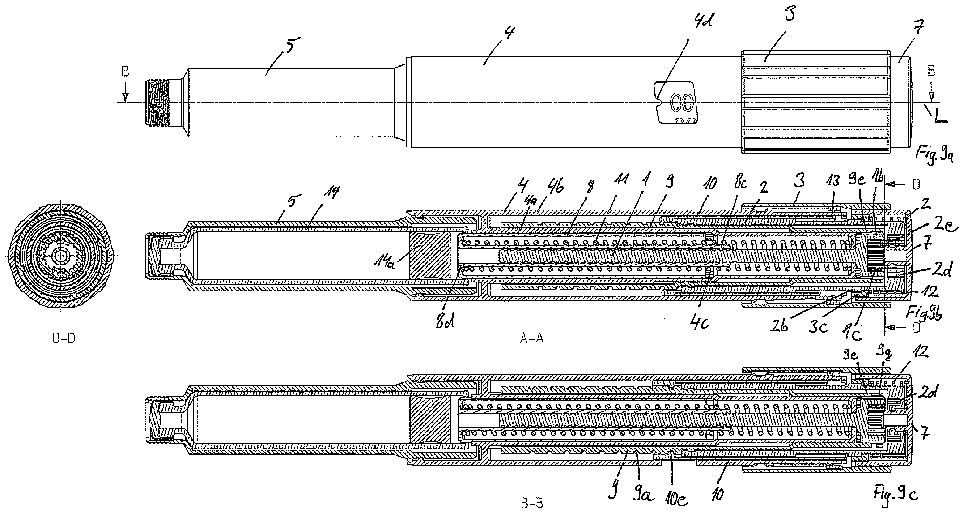

Obgleich in den Figuren 6 bis 7c keinen Begrenzer 13 gezeigt wird, kann ein solcher in einer hierin beschriebenen Art und Weise vorgesehen sein.
Eine dritte Ausführungsform einer Antriebs - und Dosiervorrichtung wird in den Figuren 8a bis 9c dargestellt. Im Folgenden werden die Merkmale beschrieben, mit denen sich diese Ausführungsform von der ersten unterscheidet, so dass im Übrigen auf die Beschreibung zu den Figuren 1 bis 5 verwiesen wird. Gleiche Bezugszeichen bezeichnen zumindest funktionell gleichartige Teile.

Die Antriebs- und Dosiervorrichtung unterscheidet sich insbesondere dadurch von der ersten Ausführungsform, dass ein Signalerzeugungsmechanismus 2e, 1c zwischen Kupplungsglied 2 und Rotationsglied 1 für die Signalisierung der Dosiseinstellung und ein weiterer Signalerzeugungsmechanismus 1b, 9g zwischen dem Rotationsglied 1 und dem Lagerelement 9 für die Signalisierung der Produktausschüttung angeordnet sind. Der Signalerzeugungsmechanismus 2e, 1c umfasst insbesondere das Rastglied 2e der Kupplungshülse 2 und die Innenverzahnung 1c des Rotationsglieds 1. Der Signalerzeugungsmechanismus 9g, 1b umfasst insbesondere das Rastglied 9g des Lagerelements 9 und die Verzahnung, d.h. die Kupplungsstruktur 1b des Rotationsglieds 1.

An dem Außenumfang des Kopfs des Rotationsglieds 1 sind parallel zur Längsachse L Zähne, die als zweite Kupplungsstruktur 1b dienen, angeordnet. An einem Innenumfang des Kopfs, der von dem Außenumfang umgeben wird, ist eine umlaufende Innenverzahnung 1c angeordnet, in die mindestens ein Rastglied 2e der Kupplungshülse 2 eingreift, vorzugsweise zumindest bei unbetätigtem Betätigungsglied 7, insbesondere sowohl bei betätigtem als auch bei unbetätigtem Betätigungsglied 7.

Eine Drehung des hülsenförmigen Kupplungsglieds 2 relativ zu dem Rotationsglied 1, wie z.B. bei der Dosiseinstellung, d.h. Drehung des Dosiseinstellglieds 3 bei unbetätigtem Betätigungselement 7, bewirkt, dass die das mindestens eine Rastglied 2e über die Innenverzahnung 1c rastet und dabei das akustische und/oder taktile Signal bei der Dosiseinstellung erzeugt.

Das Lagerelement 9 weist insbesondere an seinem proximalen Ende ein Rastglied 9g auf, welches an einem elastischen Rastarm gebildet ist und in die zweite Kupplungsstruktur 1b eingreift, vorzugsweise zumindest bei betätigtem Betätigungsglied 7, insbesondere sowohl bei betätigtem als auch bei unbetätigtem Betätigungsglied 7.

Bei betätigtem Betätigungsglied 7, d.h. wenn der Eingriff der ersten Kupplungsstruktur 9e von der zweiten Kupplungsstruktur 1b gelöst ist, kann das Rotationsglied 1 relativ zu dem Gehäuse 4 und dem Lagerelement 9 rotieren, wodurch die Zähne der zweiten Kupplungsstruktur 1b über das mindestens eine Rastglied 9g des Lagerelements 9 rasten und dabei das akustische und/oder taktile Signal bei der Produktausschüttung erzeugen.

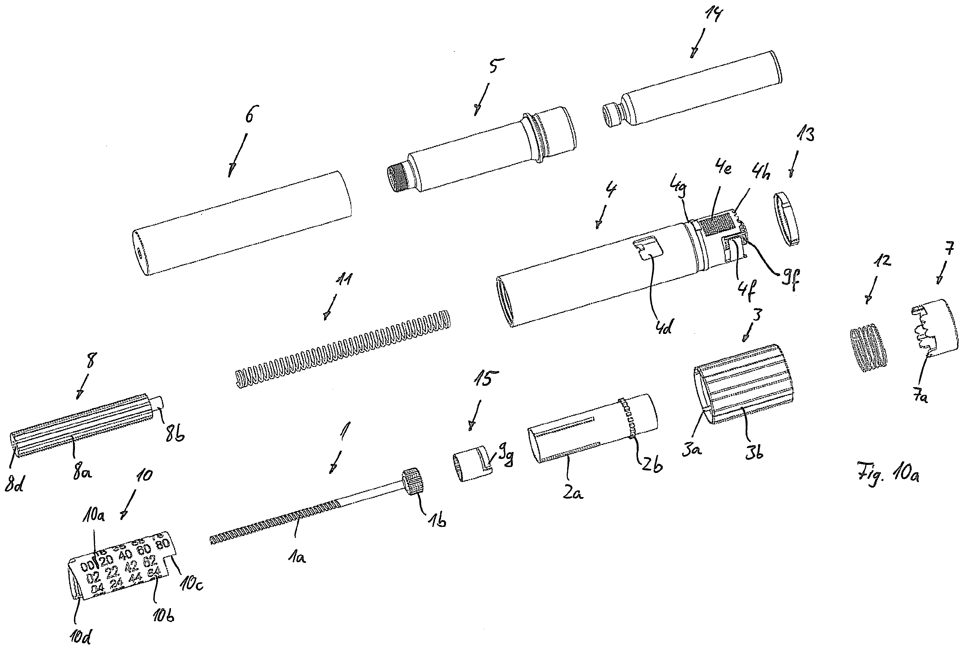

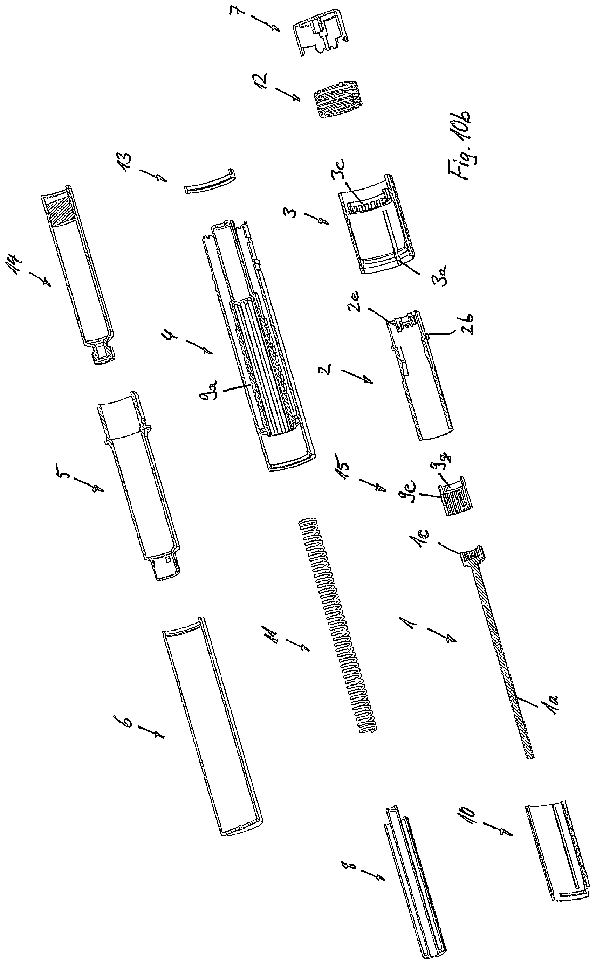

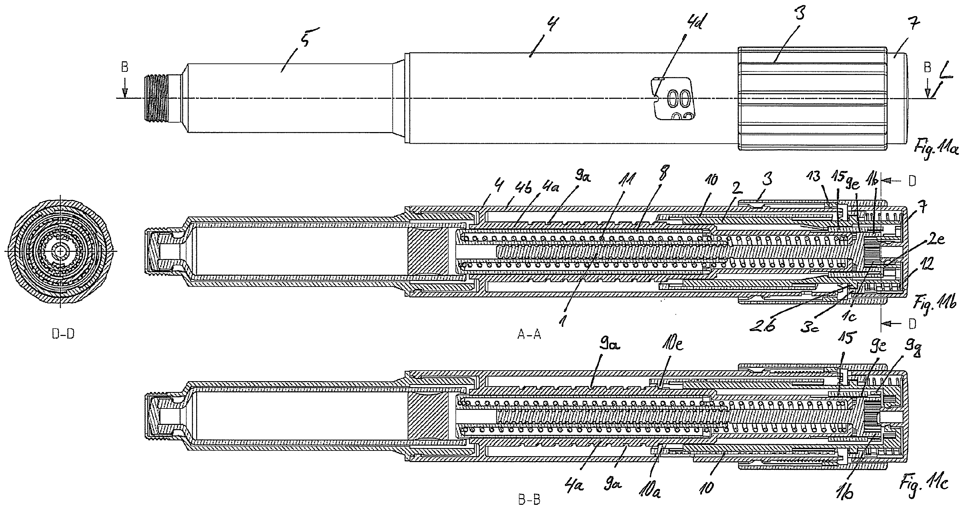

Eine vierte Ausführungsform einer Antriebs - und Dosiervorrichtung wird in den Figuren 10a bis 11c dargestellt. Im Folgenden werden die Merkmale beschrieben, mit denen sich diese Ausführungsform von ersten unterscheidet, so dass im Übrigen auf die Beschreibung zu den Figuren 1 bis 5 verwiesen wird. Gleiche Bezugszeichen bezeichnen zumindest funktionell gleichartige Teile.

Die Antriebs- und Dosiervorrichtung unterscheidet sich insbesondere dadurch von der ersten Ausführungsform, dass das Lagerelement 9, in welches die das Dosisanzeigeelement 10 mit einem Gewinde eingreift, fehlt und ein Signalerzeugungsmechanismus 2e, 1c zwischen dem Kupplungsglied 2 und dem Rotationsglied 1 für die Signalisierung der Dosiseinstellung und ein weiterer Signalerzeugungsmechanismus 9g, 1b zwischen dem Rotationsglied 1 und einer Schalthülse 15 für die Signalisierung der Produktausschüttung angeordnet sind. Der Signalerzeugungsmechanismus 2e, 1c umfasst insbesondere das Rastglied 2e des Kupplungsglieds 2 und die Innenverzahnung 1c des Rotationsglieds 1. Der Signalerzeugungsmechanismus 9g, 1b umfasst insbesondere das Rastglied 9g des Lagerelements 9 und die Außenverzahnung, d.h. die zweite Kupplungsstruktur 1b des Rotationsglieds 1.

Das Dosisanzeigeelement 10 ist in einem Gewindeeingriff mit dem Außengewinde 9a, das an der Innenhülse 4a des Gehäuses 4 gebildet ist. Dies bewirkt, dass das Dosisanzeigeelement 10 an dem Gehäuse 4 hin- und her schraubbar, jedoch nicht mehr zusätzlich zu der Schraubbewegung relativ zu dem Gehäuse 4 oder der Zeigeeinrichtung 4d axialverschiebbar ist.

Die Aufgabe der Kupplung und der Erzeugung des Signals bei der Produktausschüttung übernimmt statt dem Lagerelement 9 eine Schalthülse 15. Die Schalthülse 15 ist relativ zu dem Gehäuse 4 drehfest aber entlang der Längsachse L verschiebbar angeordnet. Die Schalthülse 15 umgibt vorzugsweise die Innenhülse 4a des Gehäuses 4, wobei insbesondere die Kupplungshülse 2 die Schalthülse 15 umgibt. Die Schalthülse 15 ist in einem Eingriff mit dem Gehäuse 4, insbesondere der Innenhülse 4a, der eine Längsbewegung der Schalthülse 15 relativ zu dem Gehäuse 4 zulässt, aber eine Drehbewegung verhindert. Der Eingriff kann durch eine Längsführung 9f zwischen der Schalthülse 15 und der Innenhülse 4a gebildet werden.

Die Schalthülse 15 weist insbesondere an ihrem proximalen Ende das Rastglied 9g auf, welches an einem elastischen Rastarm gebildet ist und in zweite Kupplungsstruktur 1b eingreift, vorzugsweise zumindest bei betätigtem Betätigungsglied 7, insbesondere sowohl bei betätigtem als auch bei unbetätigtem Betätigungsglied 7 und zur Erzeugung eines akustischen oder taktilen Signals, wie z. B. eines hör- und fühlbaren Klicks bei der Produktausschüttung dient.

Das Kupplungsglied 2 ist axialfest mit der Schalthülse 15 und relativ zu der Schalthülse 15 drehbar verbunden. Hierzu umgreift das Kupplungsglied 2 die Schalthülse 15 an ihren in Längsrichtung weisenden Stirnseiten mittels mindestens einer sich über den Innenumfang des Kupplungsglieds 2 erstreckenden Abragung. Die Schalthülse 15 wird somit - wie das Lagerelement 9 - durch die Verschiebung des Kupplungsglieds 2 mitgenommen.

An dem Außenumfang des Kopfs des Rotationsglieds 1 sind parallel zur Längsachse L Zähne 1b, die als zweite Kupplungsstruktur dienen angeordnet. An einem Innenumfang des Kopfs, der von dem Außenumfang umgeben wird, ist eine umlaufende Innenverzahnung 1c angeordnet, in die mindestens ein Rastglied 2e der Kupplungshülse 2 eingreift, vorzugsweise zumindest bei unbetätigtem Betätigungsglied 7, insbesondere sowohl bei betätigtem als auch bei unbetätigtem Betätigungsglied 7.

Eine Drehung des hülsenförmigen Kupplungsglieds 2 relativ zu dem Rotationsglied 1, wie z.B. bei der Dosiseinstellung, d.h. Drehung des Dosiseinstellglieds 3 bei unbetätigtem Betätigungselement 7, bewirkt, dass das mindestens eine Rastglied 2e über die Innenverzahnung 1c rastet und dabei das akustische und/oder taktile Signal bei der Dosiseinstellung erzeugt.

Bei betätigtem Betätigungsglied 7, d.h. wenn der Eingriff der ersten Kupplungsstruktur 9e von der zweiten Kupplungsstruktur 1b gelöst ist, kann das Rotationsglied 1 relativ zu dem Gehäuse 4 und dem Lagerelement 9 rotieren, wodurch die Zähne der zweiten Kupplungsstruktur 1b über das mindestens eine Rastglied 9g der Schalthülse 15 rasten und dabei das akustische und/oder taktile Signal bei der Produktausschüttung erzeugen.

Eine Drehung des Kupplungsglieds 2 relativ zu der Schalthülse 15 bewirkt aufgrund der drehfesten Verbindung zwischen Kupplungsglied 2 und Dosisanzeigeelement 10, dass das Dosisanzeigeelement 10 ebenfalls gedreht und aufgrund des Gewindeeingriffs in das Gewinde 9a an der Innenhülse 4a entlang geschraubt wird, insbesondere zusätzlich zu den aufgrund der Rastglieder 2e erzeugten Klickgeräuschen.

Eine Betätigung des Betätigungsglieds 7 bewirkt neben seiner axialen Verschiebung relativ zu dem Dosierglied 3 ebenfalls die axiale Verschiebung der Schalthülse 15 entlang der Längsachse L. Die Feder 12 stützt sich mit ihrem distalen Ende vorzugsweise an dem Dosierglied 3 und mit ihrem proximalen Ende vorzugsweise an dem Betätigungsglied 7 ab. Die Feder 12 ist vorzugsweise eine Schrauben- oder Wendelfeder, welche als Druckfeder wirkt.

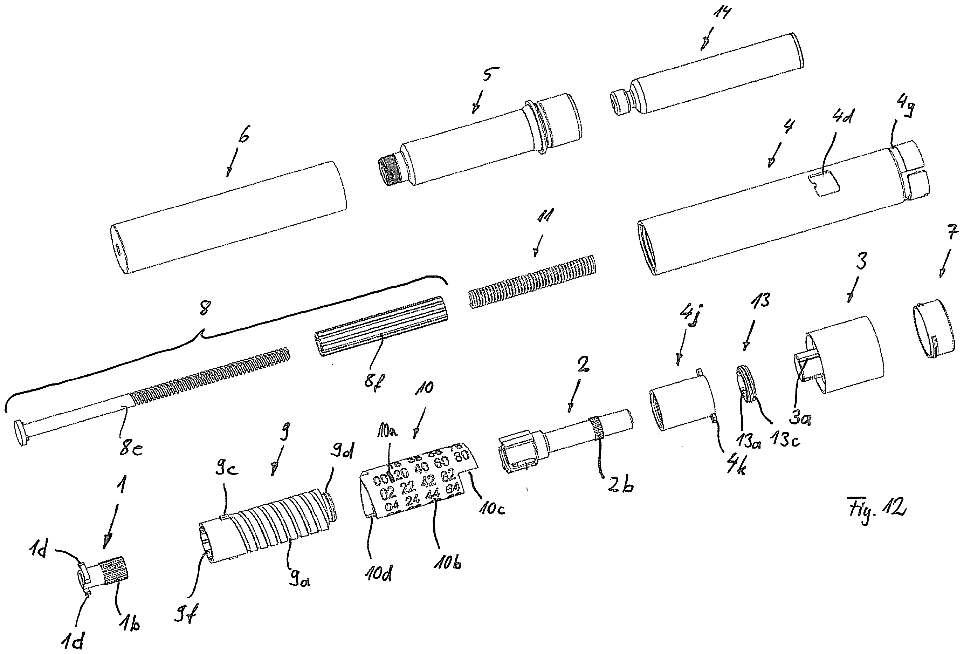

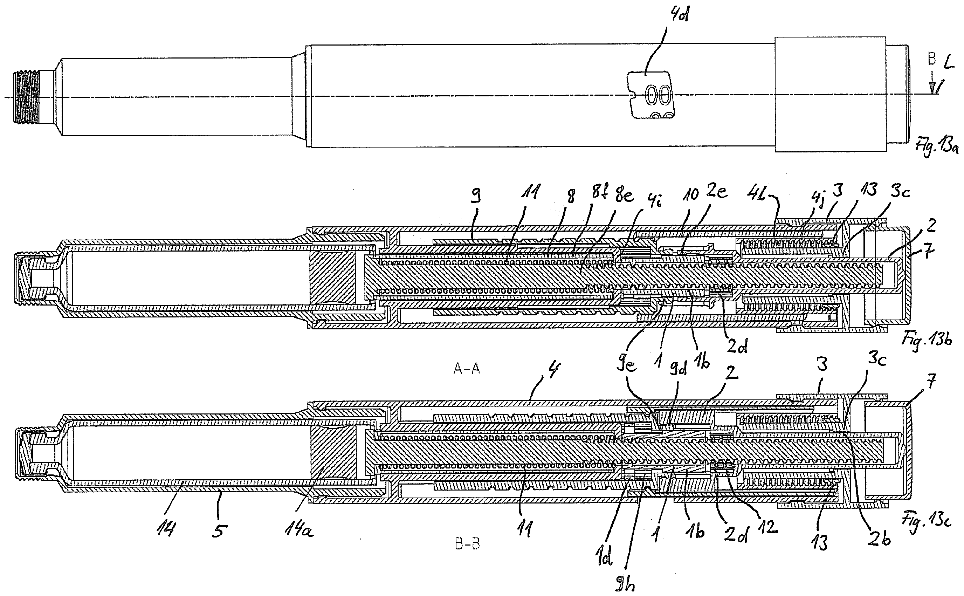

Eine fünfte Ausführungsform einer Antriebs - und Dosiervorrichtung wird in den Figuren 12 bis 13c dargestellt. Im Folgenden werden die Merkmale beschrieben, mit denen sich diese Ausführungsform von der ersten unterscheidet, so dass im Übrigen auf die Beschreibung zu den Figuren 1 bis 5 verwiesen wird. Gleiche Bezugszeichen bezeichnen zumindest funktionell gleichartige Teile.

Die Ausführungsform aus den Figuren 12 bis 13c unterscheidet sich insbesondere dadurch von der ersten Ausführungsform, dass das Rotationsglied 1 eine Gewindemutter aufweist oder ist, das Vortriebsglied 8 eine Gewindestange 8e aufweist oder ist, ein Signalerzeugungsmechanismus 2e, 1b zwischen dem Kupplungsglied 2 und dem Rotationsglied 1 für die Signalisierung der Dosiseinstellung und ein weiterer Signalerzeugungsmechanismus 1d, 9h zwischen dem Rotationsglied 1 und dem Lagerelement 9 für die Signalisierung der Produktausschüttung angeordnet sind. Ferner ist der Begrenzer 13 etwas anders ausgestaltet und die Feder 12 stützt sich mit ihrem proximalen Ende an dem Kupplungsglied 2 und ihrem distalen Ende an dem Rotationsglied 1 ab. Der Signalerzeugungsmechanismus 2e, 1b umfasst insbesondere das Rastglied 2e des Kupplungsglieds 2 und die Verzahnung, d.h. die zweite Kupplungsstruktur 1b des Rotationsglieds 1. Der Signalerzeugungsmechanismus 1d, 9h umfasst insbesondere ein Rastglied 1d des Rotationsglieds 1 und eine Innenverzahnung 9h des Lagerelements 9.

Das Gehäuse 4, insbesondere die Innenhülse 4a ist in einem drehfesten Eingriff mit einem Vortriebsglied 8, welches auch als Stößel bezeichnet werden kann. Das Vortriebsglied 8 weist eine Gewindestange 8e auf, welche in diesem Beispiel ein Außengewinde aufweist, welches in ein Innengewinde des als Gewindemutter ausgestalteten Rotationsglieds 1 eingreift.

Die Feder 11 stützt sich mit ihrem distalen Ende an einem Ringsteg des Vortriebsglieds 8 ab, der die Gewindestange 8e und die Außenhülse des Vortriebsglieds 8 verbindet. Die Feder 11 stützt sich mit ihrem proximalen Ende an dem von dem Gehäuse 4 gebildeten und nach innen ragenden Ringsteg ab, welcher das Widerlager 4i bildet.

Das Rotationsglied 1 ist als Gewindemutter ausgebildet, die das Innengewinde bildet, und weist über seinen Außenumfang parallel zur Längsachse L Zähne auf, die als zweite Kupplungsstruktur 1b dienen. An dem distalen Ende des Rotationsglieds 1 ist eine ringförmige, vom Durchmesser her verkleinerte Reibfläche angeordnet, die in einem Kontakt mit dem nach innen ragenden, das Widerlager 4i bildenden Ringsteg des Gehäuses 4 ist. Durch den verringerten Durchmesser der ringförmigen Reibfläche wird der Angriffspunkt der resultierenden Reibkraft näher zur Längsachse L hin verschoben, wodurch das Reibmoment zwischen Rotationsglied 1 und Gehäuse 4 herabgesetzt wird.

Das Kupplungsglied 2 weist das Rastglied 2e auf, welches in eine über den Umfang des Rotationsglieds 1 gebildete Verzahnung, insbesondere die Kupplungsstruktur 1b eingreift. Eine Drehung des hülsenförmigen Kupplungsglieds 2 relativ zu dem Rotationsglied 1 bewirkt, dass die Rastglieder 2e über die zweite Kupplungsstruktur 1b rasten und dabei das akustische und/oder taktile Signal während der Dosiseinstellung erzeugen.

Das Rotationsglied 1 weist an seinem distalen Ende oder distal der Kupplungsstruktur 1b das Rastglied 1d auf, welches in die über den Innenumfang des Lagerelements 9 gebildete Verzahnung 9h eingreift. Die Drehung des Rotationsglieds 1 relativ zu dem Lagerelement 9 bewirkt, dass das Rastglied 1 d über die Verzahnung 9h rastet und dabei das akustische und /oder taktile Signal während der Produktausschüttung erzeugt.

Das Lagerelement 9 weist an seinem proximalen Ende eine über seinen Umfang erstreckte ringförmige Abragung 9d auf. Das Kupplungsglied 2 ist axialfest mit dem Lagerelement 9 und relativ zu dem Lagerelement 9 drehbar verbunden. Hierzu greift das Kupplungsglied 2 mittels einer Ringnut 2c in die Abragung 9d ein.

Das Betätigungselement 7 ist relativ zu dem Dosierglied 3 frei drehbar oder drehfest, aber zumindest axial verschiebbar. Zwischen Betätigungsglied 7 und Kupplungsglied 2 ist ein Drehlager gebildet, das in dem gezeigten Beispiel durch eine auf der Drehachse L des Kupplungsglieds 2 angeordnete in etwa punktförmige Auflagefläche gebildet wird. Alternativ kann ein Gleitlager in der Gestalt eines Gleitrings, wie z.B. aus Teflon, oder ein Wälzlager, wie z.B. ein Axialkugellager als Drehlager dienen.

Der Dosisbegrenzer 13, in diesem Beispiel in der Gestalt eines Rings, weist ein Außengewinde 14c, das in ein Innengewinde 41 des Gehäuses 4 eingreift, und an seinem Innenumfang ein Eingriffsglied 13a, welches in eine Längsführung 3a des Dosierglieds 3 verdrehsicher eingreift, auf. Hierdurch ist der Begrenzer 13 durch Drehen des Dosierglieds 3 relativ zu dem Gehäuse 4 schraubbar. Das Innengewinde 41 des Gehäuses 4 wird von einer mit der Außenhülse 4b dreh- und axialfest über Verbindungsstege 4k verbundenen Begrenzerinnenhülse 4j gebildet. Die Längsführung 3a wird an einem Außenumfang einer Dosiergliedinnenhülse, die dreh- und axialfest mit der vom Verwender greifbaren Dosiergliedaußenhülse verbunden ist, gebildet. Die Dosiergliedinnenhülse ist innerhalb der Begrenzerinnenhülse 4j angeordnet, wobei der Begrenzer zwischen der Dosiergliedinnenhülse und der Begrenzerinnenhülse 4j angeordnet ist.

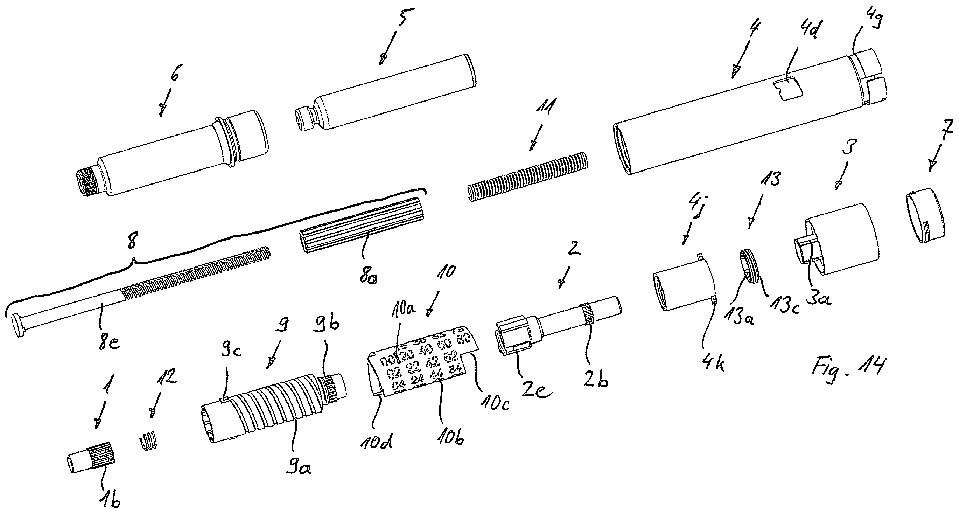

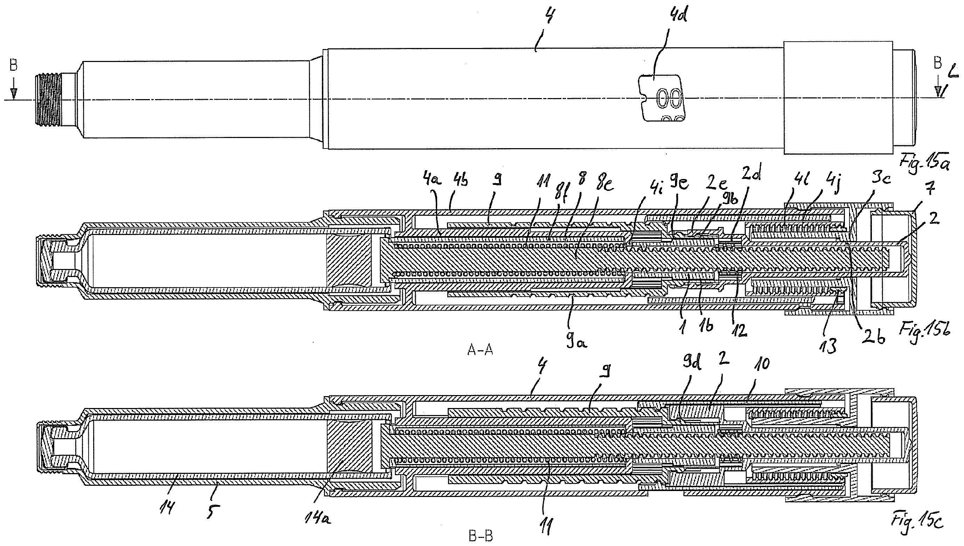

Die in den Figuren 14 bis 15 c gezeigte sechste Ausführungsform entspricht im Wesentlichen der fünften Ausführungsform, wobei der Signalerzeugungsmechanismus 2e, 9b vom Prinzip her so aufgebaut ist, wie bei der ersten Ausführungsform. Der Signalerzeugungsmechanismus 2e, 9b ist zwischen dem Kupplungsglied 2 und dem Lagerelement 9 für die Signalisierung der Dosiseinstellung und der Produktausschüttung angeordnet und umfasst insbesondere das Rastglied 2e und die Verzahnung 9b. Das Lagerelement 9 weist die sich über den Umfang erstreckende Verzahnung 9b auf. Das Kupplungsglied 2 weist das in die Verzahnung 9b eingreifende Rastglied 2e auf.

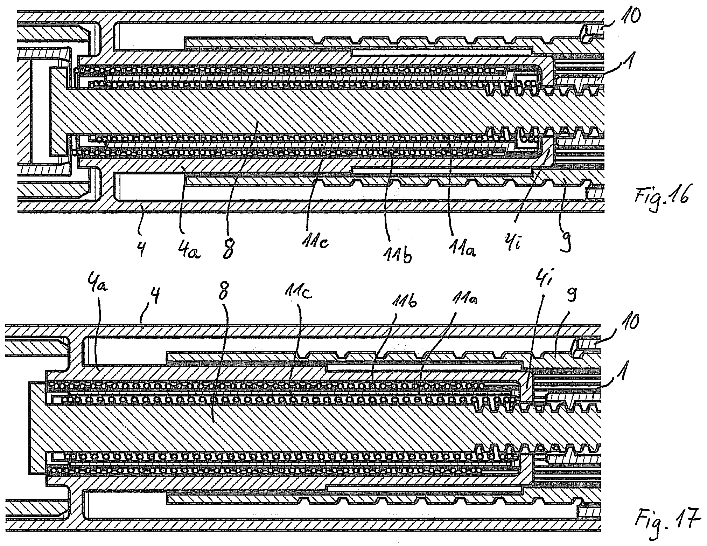

In Figur 16 wird eine Modifikation für die hierin beschriebenen Ausführungsformen dargestellt, die zwei als Druckfedern wirkende Ausschüttfedern 11a, 11b umfasst, die in Serie geschaltet sind. Die erste Ausschüttfeder 11a stützt sich mit ihrem distalen Ende an dem Vortriebsglied 8 und mit ihrem proximalen Ende an einem Zwischenglied 11c ab. Die zweite Ausschüttfeder 11b stützt sich mit ihrem distalen Ende an dem Zwischenglied 11c und mit ihrem proximalen Ende an dem Widerlager 4i ab. Die zweite Ausschüttfeder 11b ist konzentrisch zu und innerhalb der ersten Ausschüttfeder 11a angeordnet. Zwischen der ersten Ausschüttfeder 11a und der zweiten Ausschüttfeder 11b ist das hülsenförmige Zwischenglied 11c angeordnet, das insbesondere an seinem distalen Ende eine Abstützfläche für die zweite Ausschüttfeder 11b und insbesondere an seinem proximalen Ende eine Abstützfläche für die erste Ausschüttfeder 11a bildet.

In Figur 17 wird eine Modifikation für die hierin beschriebenen Ausführungsformen dargestellt, die zwei als Druckfedern wirkende Ausschüttfedern 11a, 11b umfasst, die parallel geschaltet sind. Die erste Ausschüttfeder 11a und die zweite Ausschüttfeder 11b stützen sich jeweils mit ihrem distalen Ende an dem Vortriebsglied 8 und mit ihrem proximalen Ende an dem Widerlager 4i ab. Die zweite Ausschüttfeder 11b ist konzentrisch zu und innerhalb der ersten Ausschüttfeder 11a angeordnet. Zwischen der ersten Ausschüttfeder 11a und der zweiten Ausschüttfeder ist das hülsenförmige Zwischenglied 11c angeordnet, welches verhindert, dass sich die beiden Ausschüttfedern 11a, 11b ineinander verhaken können.

**Bezugszeichenliste**

| | | | |
|---|---|---|---|
| 1 | Rotationsglied / Gewindestange / Gewindemutter | 8d | distales Ende |
| | | 8e | Gewindestange |
| 1a | Außengewinde | | |
| 1b | zweite Kupplungsstruktur / Zähne | 9 | Lagerelement |
| 1c | Verzahnung / Innenverzahnung | 9a | Außengewinde |
| 1d | Rastglied | 9b | Verzahnung / Außenverzahnung |
| | | 9c | Maximaldosisgegenanschlag |
| 2 | Kupplungsglied / Anzeigekupplung | 9d | ringförmige Abragung |
| 2a | Längsführung / Längsrippe | 9e | erste Kupplungsstruktur / Zähne |
| 2b | Kupplungsstruktur | 9f | Längsführung |
| 2c | Ringnut | 9g | Rastglied |
| 2d | dritte Kupplungsstruktur | 9h | Verzahnung / Innenverzahnung |
| 2e | Rastglied | | |
| | | 10 | Dosisanzeigeelement / Dosisanzeigetrommel |
| 3 | Dosierglied / Dosierknopf / Dosiseinstellglied | | |
| | | 10a | Markierung |
| 3a | Längsführung | 10b | Dosisskala |
| 3b | Griffstruktur | 10c | Nulldosisanschlag |
| 3c | Kupplungsstruktur | 10d | Maximaldosisanschlag |
| | | 10e | Innengewinde |
| 4 | Gehäuse | | |
| 4a | Innenhülse | 11 | Feder / Ausschüttfeder |
| 4b | Außenhülse | 11a | erste Ausschüttfeder |
| 4c | Längsführung | 11b | zweite Ausschüttfeder |
| 4d | Zeigeeinrichtung / Fenster | 11c | Zwischenglied |
| 4e | Gewinde / Außengewinde | | |
| 4f | Nulldosisgegenanschlag | 12 | Feder / Rücksetz- oder |
| 4g | Ringnut | | Kupplungsfeder |
| 4h | Verzahnung / Stirnverzahnung | | |
| 4i | Widerlager | 13 | Begrenzer / Dosisbegrenzer |
| 4j | Begrenzerinnenhülse | 13a | Eingriffsglied |
| 4k | Verbindungssteg | 13b | Gewinde / Innengewinde |
| 4l | Gewinde / Innengewinde | | |
| | | 14 | Produktbehälter / Karpule |
| 5 | Produktbehälteraufhahme | 14a | Kolben |
| | | 14b | Septum |
| 6 | Schutzkappe | | |
| | | 15 | Schalthülse |
| 7 | Betätigungsglied / Betätigungsknopf | | |
| 7a | Verzahnung / Stirnverzahnung | L | Längsachse / Drehachse |
| | | | |
| 8 | Vortriebsglied / Stößel | | |
| 8a | Längsrippe | | |
| 8b | Innenhülse | | |
| 8c | Gewinde / Innengewinde | | |

## Patentansprüche

1. Antriebs- und Dosiervorrichtung für eine Injektionsvorrichtung zur Verabreichung eines flüssigen Produkts, insbesondere eines Medikaments, wobei mit der Antriebs- und Dosiervorrichtung wiederholt zu verabreichende Produktdosen einstellbar sind, umfassend:
a) ein Gehäuse (4) und ein vom Verwender der Antriebs- und Dosiervorrichtung greifibares Dosierglied (3), welches zur Einstellung einer zu verabreichenden Produktdosis relativ zu dem Gehäuse (4) drehbar ist oder gedreht wird,
b) ein vom Verwender der Antriebs- und Dosiervorrichtung betätigbares Betätigungsglied (7), das zur Ausschüttung der eingestellten Produktdosis relativ zu dem Dosierglied (3) verschiebbar ist oder verschoben wird,
c) ein Vortriebsglied (8), dessen distales Ende (8d) vorgesehen ist, auf einen Kolben (14a) eines an der Antriebs- und Dosiervorrichtung befestigten oder befestigbaren Produktbehälters (14) zu wirken,
d) ein Widerlager (4i), wobei das Vortriebsglied (8) relativ zu dem Widerlager (4i) in eine Ausschüttrichtung bewegbar ist, um eine Ausschüttung der eingestellten Produktdosis zu bewirken,
**gekennzeichnet durch**
e) eine zwischen dem Vortriebsglied (8) und dem Widerlager (4i) wirkende und im Auslieferungszustand der Vorrichtung vorgespannte Feder (11), die mit so viel Energie vorgespannt ist, dass sie die aus dem Produktbehälter (14) maximal ausschüttbare Produktmenge in mehreren Einzelausschüttungen ausschütten kann,
f) ein Rotationsglied (1), dessen Drehung relativ zu dem Gehäuse (4) bewirkt, dass die Feder (11) das Vortriebsglied (8) in Ausschüttrichtung bewegen kann,
g) und eine Kupplung (9e, 1b), die **durch** Betätigung des Betätigungsglieds (7) die Drehung des Rotationsglieds (1) relativ zu dem Gehäuse (4) freigibt und **durch** Loslassen des Betätigungsglieds (1) die Drehung des Rotationsglieds (1) relativ zu dem Gehäuse (4) blockiert.

2. Antriebs- und Dosiervorrichtung nach Anspruch 1, **dadurch gekennzeichnet, dass** die Feder (11) mindestens eine Druckfeder ist, die sich an dem Vortriebsglied (8) und dem Widerlager (4i) abstützt, oder eine Torsionsfeder ist, die sich an dem Rotationsglied (1) und dem Widerlager (4i) abstützt.

3. Antriebs- und Dosiervorrichtung nach einem der vorhergehenden Ansprüche **dadurch gekennzeichnet, dass** das Widerlager (4i) durch das Gehäuse (4) oder einem gehäusefesten Element gebildet wird und/oder dass das Vortriebsglied (8) an dem Gehäuse (4) oder dem gehäusefesten Element geführt ist

4. Antriebs- und Dosiervorrichtung nach einem der vorhergehenden Ansprüche, **dadurch gekennzeichnet, dass** die Feder (11) das Vortriebsglied (8) antreibt und das Vortriebsglied (8) das Rotationsglied (1) antreibt.

5. Antriebs- und Dosiervorrichtung nach einem der vorhergehenden Ansprüche, **dadurch gekennzeichnet, dass** das Rotationsglied (1) eine Gewindestange und das Vortriebsglieds (8) eine Gewindemutter aufweisen und das Gewinde der Gewindemutter in das Gewinde der Gewindestange eingreift, oder dass das Rotationsglied (1) eine Gewindemutter und das Vortriebsglied (8) eine Gewindestange aufweisen und das Gewinde der Gewindemutter in das Gewinde der Gewindestange eingreift.

6. Antriebs- und Dosiervorrichtung nach einem der vorhergehenden Ansprüche, **dadurch gekennzeichnet, dass** ein Dosisanzeigeelement (10) einen Anschlag (10c) aufweist, der von einem Gegenanschlag (4f) wegbewegt wird, wenn eine Dosiserhöhung vorgenommen wird, und der zu dem Gegenanschlag (4f) hinbewegt wird, wenn eine Dosisverringerung vorgenommen wird oder wenn die Vorrichtung für die Ausschüttung der eingestellten Produktdosis betätigt wird.

7. Antriebs- und Dosiervorrichtung nach einem der vorhergehenden Ansprüche, **dadurch gekennzeichnet, dass** das Dosisanzeigeelement (10) während des Einstellens der Produktdosis, d.h. bei Dosiserhöhung und Dosisverringerung, von dem Rotationsglied (1) zumindest rotatorisch entkoppelt ist und bei der Betätigung der Vorrichtung zum Ausschütten der Produktdosis so mit dem Rotationsglied (1) gekoppelt ist, dass eine Drehung des Rotationsglieds (1) bewirkt, dass das Dosisanzeigeelement (10) zu dem Gegenanschlag (4f) hinbewegt wird.

8. Antriebs- und Dosiervorrichtung nach einem der vorhergehenden Ansprüche, **dadurch gekennzeichnet, dass** das Dosierglied (3) zumindest bei der Dosiseinstellung rotatorisch von der Feder (11) entkoppelt ist, so dass die Feder (11) bei der Dosiseinstellung weder gespannt noch entspannt wird.

9. Antriebs- und Dosiervorrichtung nach einem der vorhergehenden Ansprüche, **gekennzeichnet durch** mindestens einen Signalerzeugungsmechanismus (2e, 9b; 9g, 1b; 2e, 1c; 1d, 9h), der angepasst ist, während der Dosiseinstellung oder/und der Produktausschüttung ein akustisches und/oder taktiles Signal zu erzeugen.

10. Antriebs- und Dosiervorrichtung nach einem der vorhergehenden Ansprüche, ferner umfassend ein Dosisanzeigeelement (10), über dessen Umfang eine Dosisskala (10b) angeordnet ist, und eine Zeigeeinrichtung (4d), wobei zur Einstellung der zu verabreichenden Dosis durch Drehung des Dosierglieds (3) relativ zu der Zeigeeinrichtung (4d) das Dosisanzeigeelement (10) relativ zu der Zeigeeinrichtung (4d) und um eine Drehachse (L) drehbar, ist und mittels der Zeigeeinrichtung (4d) ein Wert der Dosisskala (10b) ablesbar ist, welcher der eingestellten Dosis entspricht.

11. Antriebs- und Dosiervorrichtung nach Anspruch 10, **dadurch gekennzeichnet, dass** es zwischen der betätigten Position und der unbetätigten Position des Betätigungsglieds (7) eine Zwischenposition gibt, in der das Rotationsglied (1) sowohl drehfest mit dem Gehäuse (4) als auch drehfest mit dem Dosisanzeigeelement (10) gekoppelt ist.

12. Antriebs- und Dosiervorrichtung nach Anspruch 10 oder 11, **dadurch gekennzeichnet, dass** zwischen dem Dosiseinstellglied (3) und dem Dosisanzeigeelement (10) eine Dosierkupplung (2b, 3c) vorgesehen ist, welche das Dosiseinstellglied (2) mit dem Dosisanzeigeelement (10) drehfest koppelt, wenn das Betätigungsglied (7) unbetätigt ist, und rotatorisch entkoppelt, wenn das Betätigungsglied (7) betätigt ist.

13. Antriebs- und Dosiervorrichtung nach einem der Ansprüche 10 bis 12, **gekennzeichnet durch** eine weitere Kupplung (1b, 2b), die bewirkt, dass das Dosisanzeigeelement (10) relativ zu dem Rotationsglied (1) drehbar ist, wenn das Betätigungsglied (7) unbetätigt ist, und das Dosisanzeigeelement (10) relativ zu dem Rotationsglied (1) drehfest ist, wenn das Betätigungsglied (7) betätigt ist.

14. Antriebs- und Dosiervorrichtung, nach einem der vorhergehenden Ansprüche, für eine Injektionsvorrichtung zur Verabreichung eines flüssigen Produkts, insbesondere eines Medikaments, wobei mit der Antriebs- und Dosiervorrichtung eine zu verabreichende Produktdosis einstellbar ist, umfassend:
a) ein Gehäuse (4),
b) ein Dosisanzeigeelement (10), über dessen Umfang eine Dosisskala (10b) angeordnet ist,
c) eine Zeigeeinrichtung (4d) und ein vom Verwender der Antriebs- und Dosiervorrichtung greifbares Dosierglied (3), wobei zur Einstellung der zu verabreichenden Dosis durch Drehung des Dosierglieds (3) relativ zu der Zeigeeinrichtung (4d) das Dosisanzeigeelement (10) relativ zu der Zeigeeinrichtung (4d) und um eine Drehachse (L) drehbar ist und mittels der Zeigeeinrichtung (4d) ein Wert der Dosisskala (10b) ablesbar ist, welcher der eingestellten Dosis entspricht,
d) ein Lagerelement (9), mit dem das Dosisanzeigeelement (10) in einem Eingriff ist, der die Dreh- oder Schraubbewegung des Dosisanzeigeelements (10) relativ zu der Zeigeeinrichtung (4d) bewirkt,
**dadurch gekennzeichnet, dass**
e) das Lagerelement (9) zusammen mit dem Dosisanzeigeelement (10) relativ zu dem Gehäuse (4) und entlang der Drehachse (L) verschiebbar ist.

15. Antriebs- und Dosiervorrichtung nach dem vorhergehenden Anspruch, **dadurch gekennzeichnet,**
**dass** das Lagerelement (9) zusammen mit dem Dosisanzeigeelement (10) relativ zu der Zeigeeinrichtung (4d), dem Gehäuse (4) und entlang der Drehachse (L) verschiebbar ist,
oder
**dass** das Lagerelement (9) zusammen mit dem Dosisanzeigeelement (10) und der Zeigeeinrichtung (4d) relativ zu dem Gehäuse (4) und entlang der Drehachse (L) verschiebbar ist.

## Claims

1. Drive and dosing device for an injection device for administering a liquid product, in particular a medicament, wherein product doses to be administered repeatedly can be set with the drive and dosing device, comprising:
a) a housing (4) and a dosing member (3), to be held by the user of the drive and dosing device, which is rotatable or is rotated relative to the housing (4) for setting a product dose to be administered,
b) an actuating member (7), to be actuated by the user of the drive and dosing device, which is displaceable or is displaced relative to the dosing member (3) for dispensing the set product dose,
c) an advancing member (8), the distal end (8d) of which is envisaged to act upon a piston (14a) of a product container (14) fixed to or fixable to the drive and dosing device,
d) a counter bearing (4i), wherein the advancing member (8) can be moved relative to the counter bearing (4i) in a dispensing direction, for effecting a dispensing of the set product dose,
**characterised by**
e) a spring (11), acting between the advancing member (8) and the counter bearing (4i) and pre-tensioned in the delivered condition of the device, pre-tension with so much energy that it can dispense the maximum product quantity to be dispensed from the product container (14) in several individual dispensing processes,
f) a rotation member (1), the turning of which relative to the housing (4) effects that the spring (11) can move the advancing member (8) in the dispensing direction,
g) and a coupling (9e, 1b), which releases the turning of the rotation member (1) relative to the housing (4) through actuating the actuating member (7), and blocks the turning of the rotation member (1) relative to the housing (4) through releasing the actuating member (7).

2. Drive and dosing device according to claim 1, **characterised in that** the spring (11) is at least one pressure spring, which supports itself on the advancing member (8) and the counter bearing (4i), or a torsion spring, which supports itself on the rotation member (1) and the counter bearing (4i).

3. Drive and dosing device according to one of the preceding claims, **characterised in that** the counter bearing (4i) is formed by the housing (4) or a housing-mounted element and/or that the advancing member (8) is held on the housing (4) or the housing-mounted element.

4. Drive and dosing device according to one of the preceding claims, **characterised in that** the spring (11) drives the advancing member (8) and the advancing member (8) drives the rotation member (1).

5. Drive and dosing device according to one of the preceding claims, **characterised in that** the rotation member (1) comprises a threaded rod and the advancing member (8) a threaded nut, and the thread of the threaded nut engages the thread of the threaded rod, or that the rotation member (1) comprises a threaded nut and the advancing member (8) comprises a threaded rod, and the thread of the threaded nut engages the thread of the threaded rod.

6. Drive and dosing device according to one of the preceding claims, **characterised in that** a dose display element (10) comprises a shoulder (10c) that is moved away from a counter shoulder (4f) when a dose increase is applied, and which is moved towards the counter shoulder (4f) when a dose reduction is applied or when the device is actuated for dispensing the set product dose.

7. Drive and dosing device according to one of the preceding claims, **characterised in that** the dose display element (10) is at least rotationally uncoupled from the rotation member (1) during the setting of the product dose, i.e. during a dose increase or a dose reduction, and is coupled with the rotation member (1) during the actuation of the device for dispensing the product dose in such a way that a turning of the rotation member (1) effects that the dose display element (10) is moved towards the counter shoulder (4i).

8. Drive and dosing according to one of the preceding claims, **characterised in that** the dosing member (3) is rotationally uncoupled from the spring (11) at least during a dose adjustment, so that the spring (11) is neither tensioned nor relaxed during the dose adjustment.

9. Drive and dosing device according to one of the preceding claims, **characterised by** at least one signal generation mechanism (2e, 9b; 9g, 1b; 2e, 1c; 1d, 9h), which is adapted for generating an acoustic and/or tactile signal during a dose adjustment or/and during product dispensing.

10. Drive and dosing device according to one of the preceding claims, further comprising a dose display element (10), around the circumference of which a dosing scale (10b) is arranged, and an indicator means (4d), wherein the dose display element (10) is rotatable, relative to the indicator means (4d) and around an axis of rotation (L) by rotating the dosing member (3) relative to the indicator means (4d) for the setting the dose to be administered, and a value of the dosing scale (10b) can be read by means of the indicator means (4d), which equals the set dose.

11. Drive and dosing device according to claim 10, **characterised in that** an interim position exists between the actuated position and the non-actuated position of the actuating member (7), in which the rotation member (1) is rotation-resistibly coupled with the housing (4) as well as rotation-resistibly coupled with dose display element (10).

12. Drive and dosing device according to claim 10 or 11, **characterised in that** a dosing coupling (2b, 3c) is envisaged between the dose setting member (3) and the dose display element (10), which rotation-resistibly couples the dose setting member (2) with the dose display element (10) when the actuating member (7) is not actuated, and rotationally uncouples the same when the actuating member (7) is actuated.

13. Drive and dosing device according to one of the claims 10 to 12, **characterised by** a further coupling (1b, 2b), which effects that the dose display element (10) can be turned relative to the rotation member (1) when the actuating member (7) is not actuated, and the dose display element (10) is rotation-resistant relative to the rotation member (1) when the actuating member (7) is actuated.

14. Drive and dosing device according to one of the preceding claims, for an injection device for administering a liquid product, in particular a medicament, wherein a product dose to be administered with the drive and dosing device can be set, comprising:
a) a housing (4).
b) a dose display element (10), around the circumference of which a dosing scale (10b) is arranged,
c) an indicator means (4d) and a dosing member (3) that can be held by the user of the drive and dosing device, wherein the dose display element (10) can be turned relative to the display means (4d) and around an axis of rotation (L) by turning the dosing member (3) relative to the indicator means (4d) for setting the dose to be administered, and a value of the dosing scale (10b) can be read by means of the display means (4d), which equals the set dose,
d) a bearing element (9), with which the dose display element (10) is in an engagement that effects the turning or screwing movement of the dose display element (10) relative to the display means (4d),
**characterised in that**
e) the bearing element (9) is displaceable together with the dose display element (10) relative to the housing (4) and along the axis of rotation (L).

15. Drive and dosing device according to one of the preceding claims, **characterised in**
**that** the bearing element (9) is displaceable relative to the display means (4d), the housing (4) and along the axis of rotation (L) together with the dose display means (10),
or
**that** the bearing element (9) is displaceable relative to the housing (4) and along the axis of rotation (L) together with the dose display element (10) and the indicator means (4d).

## Revendications

1. Dispositif d'entraînement et de dosage pour un dispositif d'injection servant à administrer un produit liquide, en particulier un médicament, dans lequel des doses de produit à administrer peuvent être réglées de manière répétée avec le dispositif d'entraînement et de dosage, comprenant :
a) un boîtier (4) et un organe de dosage (3) pouvant être saisi par l'utilisateur du dispositif d'entraînement et de dosage, lequel peut être tourné ou est tourné par rapport au boîtier (4) afin de régler une dose de produit à administrer ;
b) un organe d'actionnement (7) pouvant être actionné par l'utilisateur du dispositif d'entraînement et de dosage, lequel peut être coulissé ou est coulissé par rapport à l'organe de dosage (3) afin de distribuer la dose de produit réglée ;
c) un organe de poussée (8), dont l'extrémité (8d) distale est prévue pour agir sur un piston (14a) d'un réservoir de produit (14) fixé ou pouvant être fixé au niveau du dispositif d'entraînement et de dosage,
d) un contre-palier (4i), par rapport auquel l'organe de poussée (8) peut être déplacé dans une direction de distribution afin de provoquer une distribution de la dose de produit réglée,
**caractérisé par**
e) un ressort (11) agissant entre l'organe de poussée (8) et le contre-palier (4i) et précontraint dans l'état de livraison du dispositif, lequel ressort est précontraint avec tellement d'énergie qu'il peut distribuer en plusieurs distributions individuelles la quantité de produit pouvant être distribuée au maximum à partir du réservoir de produit (14),
f) un organe de rotation (1), dont la rotation par rapport au boîtier (4) a pour effet que le ressort (11) peut déplacer l'organe de poussée (8) dans la direction de distribution,
g) et un couplage (9e, 1b), qui libère, par l'actionnement de l'organe d'actionnement (7), la rotation de l'organe de rotation (1) par rapport au boîtier (4) et qui bloque, par le relâchement de l'organe d'actionnement (1), la rotation de l'organe de rotation (1) par rapport au boîtier (4).

2. Dispositif d'entraînement et de dosage selon la revendication 1, **caractérisé en ce que** le ressort (11) est au moins un ressort de pression, qui prend appui au niveau de l'organe de poussée (8) et du contre-palier (4i), ou est un ressort de torsion, qui prend appui au niveau de l'organe de rotation (1) et du contre-palier (4i).

3. Dispositif d'entraînement et de dosage selon l'une quelconque des revendications précédentes, **caractérisé en ce que** le contre-palier (4i) est formé par le boîtier (4) ou par un élément solidaire du boîtier, et/ou **en ce que** l'organe de poussée (8) est guidé au niveau du boîtier (4) ou au niveau de l'élément solidaire du boîtier.

4. Dispositif d'entraînement et de dosage selon l'une quelconque des revendications précédentes, **caractérisé en ce que** le ressort (11) entraîne l'organe de poussée (8), et l'organe de poussée (8) entraîne l'organe de rotation (1).

5. Dispositif d'entraînement et de dosage selon l'une quelconque des revendications précédentes, **caractérisé en ce que** l'organe de rotation (1) présente une tige filetée et l'organe de poussée (8) présente un écrou fileté et le filetage de l'écrou fileté vient en prise avec le filetage de la tige filetée, ou **en ce que** l'organe de rotation (1) présente un écrou fileté et l'organe de poussée (8) présente une tige filetée et le filetage de l'écrou fileté vient en prise avec le filetage de la tige filetée.

6. Dispositif d'entraînement et de dosage selon l'une quelconque des revendications précédentes, **caractérisé en ce qu'**un élément d'affichage de dose (10) présente une butée (10c), qui est éloignée d'une butée complémentaire (4f) quand une augmentation de dose est effectuée, et qui est rapprochée de la butée complémentaire (4f) quand une réduction de dose est effectuée ou quand le dispositif est actionné en vue de la distribution de la dose de produit réglée.

7. Dispositif d'entraînement et de dosage selon l'une quelconque des revendications précédentes, **caractérisé en ce que** l'élément d'affichage de dose (10) est découplé au moins par rotation de l'organe de rotation (1) au cours du réglage de la dose de produit, en d'autres termes lors de l'augmentation de dose et de la réduction de dose, et est couplé à l'organe de rotation (1) lors de l'actionnement du dispositif aux fins de la distribution de la dose de produit de telle sorte qu'une rotation de l'organe de rotation (1) a pour effet que l'élément d'affichage de dose (10) est rapproché de la butée complémentaire (4f).

8. Dispositif d'entraînement et de dosage selon l'une quelconque des revendications précédentes, **caractérisé en ce que** l'organe de dosage (3) est découplé par rotation du ressort (11) au moins lors du réglage de dose de telle sorte que le ressort (11) est soit tendu soit détendu lors du réglage de dose.

9. Dispositif d'entraînement et de dosage selon l'une quelconque des revendications précédentes, **caractérisé par** au moins un mécanisme de génération de signaux (2e, 9b ; 9g, 1b ; 2e, 1c; 1d, 9h), qui est adapté pour générer un signal acoustique et/ou tactile au cours du réglage de dose et/ou de la distribution de produit.

10. Dispositif d'entraînement et de dosage selon l'une quelconque des revendications précédentes, comprenant en outre un élément d'affichage de dose (10), sur la périphérie duquel une graduation de dose (10b) est disposée, et un système indicateur (4d), dans lequel l'élément d'affichage de dose (10) peut être tourné par rapport au système indicateur (4d) et autour d'un axe de rotation (L) afin de régler la dose à administrer par la rotation de l'organe de dosage (3) par rapport au système indicateur (4d), et une valeur de la graduation de dose (10b) peut être lue au moyen du système indicateur (4d), laquelle correspond à la dose réglée.

11. Dispositif d'entraînement et de dosage selon la revendication 10, **caractérisé en ce qu'**il existe, entre la position actionnée et la position non actionnée de l'organe d'actionnement (7), une position intermédiaire, dans laquelle l'organe de rotation (1) est couplé aussi bien de manière solidaire en rotation au boîtier (4) que de manière solidaire en rotation à l'élément d'affichage de dose (10).

12. Dispositif d'entraînement et de dosage selon la revendication 10 ou 11, **caractérisée en ce qu'**est prévu, entre l'organe de réglage de dose (3) et l'élément d'affichage de dose (10), un couplage de dosage (2b, 3c), qui couple de manière solidaire en rotation l'organe de réglage de dose (2) à l'élément d'affichage de dose (10) quand l'organe d'actionnement (7) n'est pas actionné et le découple par rotation quand l'organe d'actionnement (7) est actionné.

13. Dispositif d'entraînement et de dosage selon l'une quelconque des revendications 10 à 12, **caractérisé par** un autre couplage (1b, 2b) qui a pour effet que l'élément d'affichage de dose (10) peut être tourné par rapport à l'organe de rotation (1) quand l'organe d'actionnement (7) n'est pas actionné, et l'élément d'affichage de dose (10) est solidaire en rotation par rapport à l'organe de rotation (1) quand l'organe d'actionnement (7) est actionné.

14. Dispositif d'entraînement et de dosage selon l'une quelconque des revendications précédentes, pour un dispositif d'injection servant à administrer un produit liquide, en particulier un médicament, dans lequel une dose de produit à administrer peut être réglée avec le dispositif d'entraînement et de dosage, comprenant :
a) un boîtier (4) ;
b) un élément d'affichage de dose (10), sur la périphérie duquel une graduation de dose (10b) est disposée,
c) un système indicateur (4d) et un organe de dosage (3) pouvant être saisi par l'utilisateur du dispositif d'entraînement et de dosage, dans lequel l'élément d'affichage de dosage (10) peut être tourné par rapport au système indicateur (4d) et autour d'un axe de rotation (L) afin de régler la dose à administrer par la rotation de l'organe de dosage (3) par rapport au système indicateur (4d) et une valeur de la graduation de dose (10b) peut être lue au moyen du système indicateur (4d), laquelle correspond à la dose réglée,
d) un élément de palier (9), avec lequel l'élément d'affichage de dose (10) est en prise, lequel provoque le déplacement par rotation ou par vissage de l'élément d'affichage de dose (10) par rapport au système indicateur (4d),
caractérisé en ce
e) l'élément de palier (9) peut être coulissé conjointement avec l'élément d'affichage de dose (10) par rapport au boîtier (4) et le long de l'axe de rotation (L).

15. Dispositif d'entraînement et de dosage selon la revendication précédente, **caractérisé en ce**
**que** l'élément de palier (9) peut être coulissé conjointement avec l'élément d'affichage de dose (10) par rapport au système indicateur (4d), au boîtier (4) et le long de l'axe de rotation (L),
ou
**que** l'élément de palier (9) peut être coulissé conjointement avec l'élément d'affichage de dose (10) et le système indicateur (4d) par rapport au boîtier (4) et le long de l'axe de rotation (L).
